(19) Europäisches Patentamt
European Patent Office
Office européen des brevets

(11) EP 0 567 966 B1

(12) EUROPÄISCHE PATENTSCHRIFT

(45) Veröffentlichungstag und Bekanntmachung des Hinweises auf die Patenterteilung:
**02.09.1998 Patentblatt 1998/36**

(21) Anmeldenummer: **93106724.3**

(22) Anmeldetag: **26.04.1993**

(51) Int. Cl.[6]: **C07D 207/26**, A61K 31/40, C07D 207/48, C07D 401/12, C07D 403/12, C07D 405/12, C07F 9/24, A61K 31/34, A61K 31/445

# (54) Cyclische Iminoderivate, Verfahren zu ihrer Herstellung und diese Verbindungen enthaltende Arzneimittel

Cyclic imino derivatives, medicaments containing these compounds and processes for the production thereof

Composés imino cycliques, médicaments les contenant et procédés pour leur préparation

(84) Benannte Vertragsstaaten:
**AT BE CH DE DK ES FR GB GR IE IT LI LU NL PT SE**

(30) Priorität: **28.04.1992 DE 4213919**

(43) Veröffentlichungstag der Anmeldung:
**03.11.1993 Patentblatt 1993/44**

(73) Patentinhaber:
**Boehringer Ingelheim Pharma KG**
**55216 Ingelheim am Rhein (DE)**

(72) Erfinder:
- **Himmelsbach, Frank, Dr.**
  **W-7951 Mittelbiberach (DE)**
- **Austel, Volkhard, Dr.**
  **W-7950 Biberach 1 (DE)**
- **Pieper, Helmut, Dr.**
  **W-7950 Biberach 1 (DE)**
- **Linz, Günter, Dr.**
  **W-7950 Biberach 1 (DE)**
- **Weisenberger, Johannes, Dr.**
  **W-7950 Biberach 1 (DE)**
- **Müller, Thomas, Dr.**
  **W-7950 Biberach 1 (DE)**

(74) Vertreter: **Laudien, Dieter**
**Boehringer Ingelheim GmbH,**
**Binger Strasse 173**
**55216 Ingelheim (DE)**

(56) Entgegenhaltungen:

| | |
|---|---|
| **EP-A- 0 194 548** | **EP-A- 0 196 184** |
| **EP-A- 0 350 437** | **EP-A- 0 397 044** |
| **EP-A- 0 409 163** | **EP-A- 0 483 667** |
| **EP-A- 0 503 548** | **EP-A- 0 525 629** |
| **EP-A- 0 528 369** | **US-A- 4 491 591** |

- **PATENT ABSTRACTS OF JAPAN vol. 014, no. 566 17. Dezember 1990**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.3/3.4

## Beschreibung

In der EP-A-0,196,184 werden u.a. 1-Hydroxy-5-biphenylalkyl-2-pyrrolidinone beschrieben, welche entzündungshemmende Eigenschaften aufweisen.

Es wurde nun gefunden, daß die 2-Pyrrolidinone der allgemeinen Formel

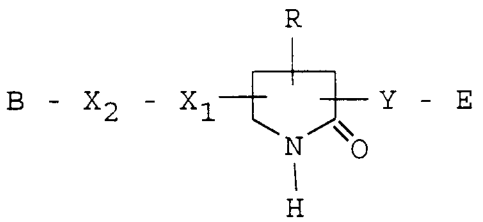
, (I)

deren Stereoisomere, deren Tautomere, deren Gemische und deren Salze, insbesondere deren physiologisch verträgliche Salze mit anorganischen oder organischen Säuren oder Basen, ebenfalls wertvolle pharmakologische Eigenschaften aufweisen, vorzugsweise jedoch aggregationshemmende Wirkungen.

Gegenstand der vorliegenden Erfindung sind somit die 2-Pyrrolidinone der obigen allgemeinen Formel I, diese Verbindungen enthaltende Arzneimittel, deren Verwendung und Verfahren zu ihrer Herstellung.

In der obigen allgemeinen Formel I bedeutet mit der Maßgabe, daß

(i) B eine $R_1$-CO-O-($R_2CR_3$)-O-CO-NH-C(=NH)- oder ($R_4$O)PO(O$R_5$)-NH-C(=NH)-Gruppe oder E eine $R_6$-CO-O-CH$R_7$-O-CO- oder $R_8$O-CO-Gruppe oder

(ii) B eine $R_1$-CO-O-($R_2CR_3$)-O-CO-NH-C(=NH)- oder ($R_4$O)PO(O$R_5$)-NH-C(=NH)-Gruppe und E eine $R_6$-CO-O-CH$R_7$-O-CO- oder $R_8$O-CO-Gruppe darstellen, in denen

$R_1$ eine Alkylgruppe mit 1 bis 4 Kohlenstoffatomen,

$R_2$ ein Wasserstoffatom oder eine Methylgruppe,

$R_3$ ein Wasserstoffatom,

$R_4$ und $R_5$, die gleich oder verschieden sein können, Methyl- oder Ethylgruppen,

$R_6$ eine Alkylgruppe mit 1 bis 4 Kohlenstoffatomen, eine Cyclohexyl- oder Phenylgruppe, eine Alkoxygruppe mit 1 bis 4 Kohlenstoffatomen oder eine Cycloalkoxygruppe mit 5 bis 7 Kohlenstoffatomen,

$R_7$ ein Wasserstoffatom oder eine Methylgruppe und

$R_8$ eine Cycloalkyl-, Cycloalkylmethyl- oder Cycloalkylethylgruppe mit jeweils 5 bis 8 Kohlenstoffatomen im Cycloalkylteil, wobei die vorstehend erwähnten Cycloalkylteile zusätzlich durch eine Alkylgruppe mit 1 bis 4 Kohlenstoffatomen oder durch eine Alkylgruppe mit 1 bis 4 Kohlenstoffatomen und 1 bis 3 Methylgruppen, durch eine Methoxy-, Ethoxy-, Dimethylamino- oder Diethylaminogruppe substituiert sein können und außerdem in den vorstehend erwähnten Cycloalkylteilen jeweils eine Methylengruppe durch ein Sauerstoffacom, durch eine Methylimino- oder Ethyliminogruppe mit der Maßgabe ersetzt sein kann, daß sich zwischen dem Ringheteroatom und dem nächsten Heteroatom mindestens 2 Kohlenstoffatome befinden,

eine Cyclohexenyl- oder Cyclohexenylmethylgruppe, wobei die vorstehend erwähnten Cyclohexenylteile zusätzlich durch eine Methylgruppe substituiert sein können, mit der Maßgabe, daß die vorstehend erwähnten Cyclohexenylteile nicht über ein Kohlenstoffatom, von dem eine Doppelbindung ausgeht, mit dem Sauerstoffatom der benachbarten -O-CO-Gruppe verknüpft sind,

eine Bicycloalkyl- oder Bicycloalkylalkylgruppe mit jeweils 6 bis 8 Kohlenstoffatomen im Bicycloalkylteil und 1 oder 2 Kohlenstoffatomen im Alkylteil, wobei die vorstehend erwähnten Bicycloalkylteile zusätzlich durch 1 bis 3 Methylgruppen substituiert sein können,

eine Bicycloalkenyl- oder Bicycloalkenylalkylgruppe mit jeweils 7 oder 8 Kohlenstoffatomen im Bicycloalkenylteil und 1 oder 2 Kohlenstoffatomen im Alkylteil, wobei die vorstehend erwähnten Bicycloalkenylteile zusätzlich durch 1 bis 3 Methylgruppen subtituiert sein können, mit der Maßgabe, daß die vorstehend erwähnten Bicycloalkenylteile nicht über ein Kohlenstoffatom, von dem eine Doppelbindung ausgeht, mit dem Sauerstoffatom der benachbarten -O-CO-Gruppe verknüpft sind,

eine Benzocycloalkylgruppe mit insgesamt 9 oder 10 Kohlenstoffatomen,

eine Alkenyl- oder Alkinylgruppe mit jeweils 3 bis 5 Kohlenstoffatomen mit der Maßgabe, daß die vorstehend erwähnten Alkenyl- oder Alkinylgruppen nicht über ein Kohlenstoffatom, von dem eine Doppel- oder Dreifachbindung ausgeht, mit dem Sauerstoffatom der benachbarten -O-CO-Gruppe verknüpft sind, oder

eine Cinnamylgruppe bedeuten,

R ein an ein Kohlenstoffatom des 2-Pyrrolidinonringes gebundenes Wasserstoffatom oder eine Methylgruppe,

B eine $HNR_{11}$-C(=NH)-Gruppe, in der $R_{11}$ ein Wasserstoffatom, eine Methoxycarbonyl-, Ethoxycarbonyl- oder Benzyloxycarbonylgruppe darstellt, eine $R_1$-CO-O-($R_2CR_3$)-O-CO-NH-C(=NH)- oder ($R_4$O)PO($OR_5$)-NH-C(=NH)-Gruppe, in denen $R_1$ bis $R_5$ wie vorstehend erwähnt definiert sind,

Y eine Methylengruppe,

E eine Carboxygruppe, eine Alkoxycarbonylgruppe mit insgesamt 2 bis 6 Kohlenstoffatomen, eine $R_6$-CO-O-$CHR_7$-O-CO- oder $R_8$O-CO-Gruppe, wobei $R_6$ bis $R_8$ wie vorstehend erwähnt definiert sind

$X_1$ eine Methylengruppe, die, sofern diese nicht an das Ringstickstoffatom des 2-Pyrrolidinonringes gebunden ist, über ein Sauerstoffatom, eine Sulfonyl-, -CO-NH- oder -$SO_2$-NH-Gruppe an die benachbarte $X_2$-Gruppe gebunden ist, wobei die Carbonyl- und Sulfonylgruppe der vorstehend erwähnten -CO-NH- und -$SO_2$-NH-Gruppen mit dem Rest $X_2$ verknüpft ist, und

$X_2$ eine Biphenylylengruppe, die durch ein Fluor- oder Chloratom, durch eine Methyl- oder Trifluormethylgruppe oder durch eine weitere Methylgruppe substituiert sein kann.

Bevorzugte Verbindungen der obigen allgemeinen Formel sind diejenigen, in denen mit der Maßgabe, daß

(i) B eine $R_1$-CO-O-($R_2CR_3$)-O-CO-NH-C(=NH)- oder ($R_4$O)PO($OR_5$)-NH-C(=NH)-Gruppe oder
E eine $R_6$-CO-O-$CHR_7$-O-CO- oder $R_8$O-CO-Gruppe oder

(ii) B eine $R_1$-CO-O-($R_2CR_3$)-O-CO-NH-C(=NH)- oder ($R_4$O)PO($OR_5$)-NH-C(=NH)-Gruppe und
E eine $R_6$-CO-O-$CHR_7$-O-CO- oder $R_8$O-CO-Gruppe darstellen, in denen

$R_1$ eine Methylgruppe,

$R_2$ ein Wasserstoffatom oder eine Methylgruppe,

$R_3$ ein Wasserstoffatom,

$R_4$ und $R_5$ jeweils eine Ethylgruppe,

$R_6$ eine tert.Butylgruppe, eine Ethoxy- oder Cyclohexyloxygruppe,

$R_7$ ein Wasserstoffatom oder eine Methylgruppe und

$R_8$ eine Cyclopentyl-, Cyclohexyl-, Cycloheptyl- oder Cyclohexylmethylgruppe bedeuten,

R ein Wasserstoffatom,

B eine $HNR_{11}$-C(=NH)-Gruppe, in der $R_{11}$ ein Wasserstoffatom, eine Methoxycarbonyl- oder Benzyloxycarbonyl-

gruppe darstellt, eine $R_1$-CO-O-($R_2CR_3$)-O-CO-NH-C(=NH)- oder ($R_4O$)PO($OR_5$)-NH-C(=NH)-Gruppe, in denen $R_1$ bis $R_5$ wie vorstehend erwähnt definiert sind,

Y eine Methylengruppe,

E eine Methoxycarbonylgruppe, eine $R_6$-CO-O-$CHR_7$-O-CO- oder $R_8$O-CO-Gruppe, wobei $R_6$ bis $R_8$ wie vorstehend erwähnt definiert sind,

$X_1$ und $X_2$ zusammen eine 4-Biphenylylenoxymethylengruppe, wobei die Gruppe B in 4'-Stellung mit der 4-Biphenylylenoxymethylengruppe verknüpft ist, und die Methylengruppe der 4-Biphenylylenoxymethylengruppe mit einem Kohlenstoffatom des 2-Pyrrolidinonringes verknüpft ist, bedeuten,
deren Stereoisomere, deren Tautomere, deren Gemische und deren Salze.

Besonders bevorzugte Verbindungen der allgemeinen Formel I sind jedoch die vorstehend erwähnten Verbindungen, in denen der Rest -Y-E in Position 3 und
der Rest B-$X_2$-$X_1$- in Position 5 des 2-Pyrrolidinonringes steht,
deren Stereoisomere, deren Tautomere, deren Gemische und deren Salze.
Als besonders bevorzugte Verbindungen seien beispielsweise folgende erwähnt:

(3S,5S)-5-[(4'-Acetoxymethyloxycarbonylamidino-4-biphenylyl)oxymethyl]-3-[(methoxycarbonyl)methyl]-2-pyrrolidinon,

(3S,5S)-5-[[4'-(1-Acetoxyethyl)oxycarbonylamidino-4-biphenylyl]oxymethyl]-3-[(methoxycarbonyl)methyl]-2-pyrrolidinon,

(3S,5S)-5-[(4'-Benzyloxycarbonylamidino-4-biphenylyl)oxymethyl]-3-[[[1-(cyclohexyloxycarbonyloxy)ethyl]oxycarbonyl]-methyl]-2-pyrrolidinon,

(3S,5S)-5-[(4'-Benzyloxycarbonylamidino-4-biphenylyl)-oxymethyl]-3-[[[1-(ethyloxycarbonyloxy)ethyl]oxycarbonyl]-methyl]-2-pyrrolidinon,

(3S,5S)-5-[(4'-Benzyloxycarbonylamidino-4-biphenylyl)-oxymethyl]-3-[(pivaloyloxymethyloxycarbonyl)methyl]-2-pyrrolidinon,

(3S,5S)-5-[(4'-Methoxycarbonylamidino-4-biphenylyl)oxymethyl]-3-[(pivaloyloxymethyloxycarbonyl)methyl]-2-pyrrolidinon,

(3S,5S)-5-[(4'-Amidino-4-biphenylyl)oxymethyl]-3-[[[1-(cyclohexyloxycarbonyloxy)ethyl]oxycarbonyl]methyl]-2-pyrrolidinon,

(3S,5S)-5-[(4'-Amidino-4-biphenylyl)oxymethyl]-3-[[[1-(ethyloxycarbonyloxy)ethyl]oxycarbonyl]methyl]-2-pyrrolidinon,

(3S,5S)-5-[(4'-Amidino-4-biphenylyl)oxymethyl]-3-[(pivaloyloxymethyloxycarbonyl)methyl]-2-pyrrolidinon,

(3S,5S)-5-[(4'-Amidino-4-biphenylyl)oxymethyl]-3-[(cyclohexyloxycarbonyl)methyl]-2-pyrrolidinon,

(3S,5S)-5-[(4'-Amidino-4-biphenylyl)oxymethyl]-3-[(cyclohexylmethyloxycarbonyl)methyl]-2-pyrrolidinon,

(3S,5S)-5-[(4'-Amidino-4-biphenylyl)oxymethyl]-3-[(cyclopentyloxycarbonyl)methyl]-2-pyrrolidinon,

(3S,5S)-5-[(4'-Amidino-4-biphenylyl)oxymethyl]-3-[(cycloheptyloxycarbonyl)methyl]-2-pyrrolidinon,

(3S,5S)-3-[(Cyclohexyloxycarbonyl)methyl]-5-[(4'-methoxycarbonylamidino-4-biphenylyl)oxymethyl]-2-pyrrolidinon und

(3S,5S)-5-[[4'-(O,O'-Diethylphosphono)amidino-4-biphenylyl]oxymethyl]-3-[(methoxycarbonyl)methyl]-2-pyrrolidinon

sowie deren Salze.

Erfindungsgemäß erhält man die neuen Verbindungen der allgemeinen Formel I nach folgenden Verfahren:

a) Zur Herstellung von Verbindungen der allgemeinen Formel I, in der B eine $R_1$-CO-O-($R_2CR_3$)-O-CO-NH-C(=NH)- oder $HNR_{11}$-C(=NH)-Gruppe darstellt, wobei $R_1$ bis $R_3$ wie eingangs erwähnt definiert sind und $R_{11}$ eine Methoxycarbonyl-, Ethoxycarbonyl- oder Benzyloxycarbonylgruppe bedeuten:
Umsetzung einer Verbindung der allgemeinen Formel

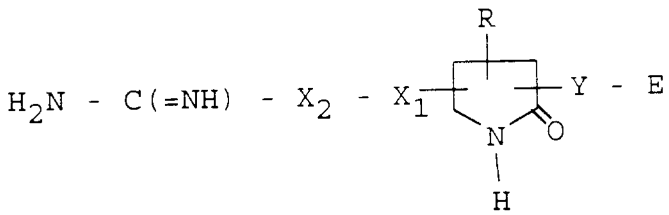
, (II)

in der

R, E, $X_1$, $X_2$ und Y wie eingangs erwähnt definiert sind, mit einer Verbindung der allgemeinen Formel

$$Z_1 - B_1 , \quad \text{(III)}$$

in der
$B_1$ eine Methoxycarbonyl-, Ethoxycarbonyl- oder Benzyloxycarbonylgruppe oder eine $R_1$-CO-O-($R_2CR_3$)-O-CO-Gruppe, in der $R_1$ bis $R_3$ wie eingangs erwähnt definiert sind, und
$Z_1$ eine nukleophile Austrittsgruppe wie ein Halogenatom oder eine Aryloxygruppe, z. B. ein Chlor- oder Bromatom oder eine p-Nitrophenoxygruppe, bedeuten.

Die Umsetzung wird zweckmäßigerweise in einem Lösungsmittel wie Tetrahydrofuran, Methylenchlorid, Chloroform, Essigester oder Dimethylformamid zweckmäßigerweiSe in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100°C, vorzugsweise jedoch bei Temperaturen zwischen -10 und 60°C, durchgeführt.

b) Zur Herstellung von Verbindungen der allgemeinen Formel I, in der E eine Alkoxycarbonylgruppe mit insgesamt 2 bis 6 Kohlenstoffatomen, eine $R_6$-CO-O-$CHR_7$-O-CO- oder $R_8$O-CO-Gruppe darstellt, wobei $R_6$ bis $R_8$ wie eingangs erwähnt definiert sind:
Umsetzung einer Verbindung der allgemeinen Formel

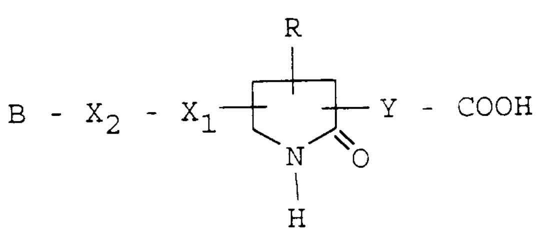
, (IV)

in der

R, B, $X_1$, $X_2$ und Y wie eingangs erwähnt definiert sind, mit einer Verbindung der allgemeinen Formel

$$Z_2 - E_1 , \quad \text{(V)}$$

in der

$E_1$ eine Alkylgruppe mit insgesamt 1 bis 5 Kohlenstoffatomen, eine $R_6$-CO-O-CHR$_7$- oder $R_8$-Gruppe darstellt, wobei $R_6$ bis $R_8$ wie eingangs erwähnt definiert sind, und

$Z_2$ eine nukleophile Austrittsgruppe wie ein Halogenatom, z. B. ein Chlor-, Brom- oder Jodatom, darstellen.

Die Umsetzung wird zweckmäßigerweise in einem Lösungsmittel wie Methylenchlorid, Tetrahydrofuran, Dioxan, Dimethylsulfoxid oder Dimethylformamid gegebenenfalls in Gegenwart eines Reaktionsbeschleunigers wie Natrium- oder Kaliumjodid und vorzugsweise in Gegenwart einer Base wie Natriumcarbonat, Kaliumcarbonat oder Natronlauge oder in Gegenwart einer tertiären organischen Base wie N-Ethyl-diisopropylamin oder N-Methylmorpholin, welche gleichzeitig auch als Lösungsmittel dienen können, oder gegebenenfalls in Gegenwart von Silberkarbonat oder Silberoxid bei Temperaturen zwischen -30 und 100°C, vorzugsweise jedoch bei Temperaturen zwischen -10 und 80°C, durchgeführt.

c) Zur Herstellung von Verbindungen der allgemeinen Formel I, in der E eine Alkoxycarbonylgruppe mit insgesamt 2 bis 6 Kohlenstoffatomen oder eine $R_8$O-CO-Gruppe darstellt, wobei $R_8$ wie eingangs erwähnt definiert ist: Veresterung einer Verbindung der allgemeinen Formel

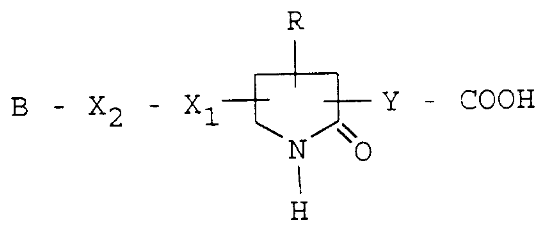

, (IV)

in der

R, B, $X_1$, $X_2$ und Y wie eingangs erwähnt definiert sind, mit einer Verbindung der allgemeinen Formel

$$HO - E_2 , \qquad (VI)$$

in der

$E_2$ eine Alkylgruppe mit 1 bis 5 Kohlenstoffatomen oder eine $R_8$-Gruppe, wobei $R_8$ wie eingangs erwähnt definiert ist, darstellt.

Die Veresterung wird zweckmäßigerweiSe in einem geeigneten Lösungsmittel oder Lösungsmittelgemisch, z. B. in einem entsprechenden Alkohol wie Methanol, Ethanol, Isopropanol oder Cyclohexanol, Methylenchlorid, Tetrahydrofuran, Dioxan, Pyridin, Toluol oder Dimethylformamid in Gegenwart eines säureaktivierenden und/oder wasserentziehenden Mittels wie Chlorwasserstoff, konz. Schwefelsäure, Thionylchlorid, Titantetrachlorid, Trimethylchlorsilan, Chlorameisensäureethylester, Carbonyldiimidazol oder N,N'-Dicyclohexyl-carbodiimid oder dessen Isoharnstoffestern, gegebenenfalls in Gegenwart eines Reaktionsbeschleunigers wie Kupferchlorid, bei Temperaturen zwischen 0 und 100°C, vorzugsweise jedoch bei Temperaturen zwischen 20°C und 80°C, durchgeführt.

d) Zur Herstellung von Verbindungen der allgemeinen Formel I, in der B eine ($R_4$O)PO(O$R_5$)-NH-C(=NH)-Gruppe darstellt, wobei $R_4$ und $R_5$ wie eingangs erwähnt definiert sind: Umsetzung einer Verbindung der allgemeinen Formel

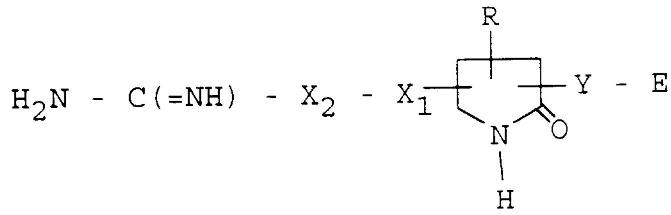 , (II)

in der

R, E, $X_1$, $X_2$ und Y wie eingangs erwähnt definiert sind, mit einer Verbindung der allgemeinen Formel

$$(R_4O)PO(OR_5)\text{-}Z_3 , \qquad (VII)$$

in der
$R_4$ und $R_5$ wie eingangs erwähnt definiert sind und $Z_3$ eine nukleophile Austrittsgruppe wie eine Cyanogruppe oder ein Halogenatom, z. B. ein Chlor- oder Bromatom, bedeuten.

Die Umsetzung wird zweckmäßigerweise in einem Lösungsmittel oder Lösungsmittelgemisch wie Tetrahydrofuran, Methylenchlorid, Chloroform, Essigester oder Dimethylformamid zweckmäßigerweise in Gegenwart einer anorganischen Base wie Natriumkarbonat, Kaliumkarbonat oder Natronlauge oder in Gegenwart einer tertiären organischen Base wie Triethylamin, N-Ethyldiisopropylamin, N-Methyl-morpholin oder Pyridin, welche gleichzeitig auch als Lösungsmittel dienen können, bei Temperaturen zwischen -30°C und 100°C, vorzugsweise jedoch bei Temperaturen zwischen -10°C und 60°C, durchgeführt.

Bei den vorstehend beschriebenen Umsetzungen können gegebenenfalls vorhandene reaktive Gruppen wie Carboxy- oder Amidinogruppen während der Umsetzung durch übliche Schutzgruppen geschützt werden, welche nach der Umsetzung wieder abgespalten werden.

Beispielsweise kommt als Schutzrest für eine Carboxylgruppe die Trimethylsilyl-, Methyl-, Ethyl-, tert.Butyl-, Benzyl- oder Tetrahydropyranylgruppe und
als Schutzrest für eine Amidinogruppe die Benzyloxycarbonylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen -10 und 100°C, vorzugsweise bei Temperaturen zwischen 0 und 60°C.

Die Abspaltung eines Benzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung eines tert.Butylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihren

aktivierten Derivaten oder Alkoholen, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Essigsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine saure Gruppe wie eine Carboxyl- oder Phosphonogruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierfür beispielsweise Natriumhydroxid, Kaliumhydroxid, Ammoniak, Cyclohexylamin, Äthanolamin, Diäthanolamin und Triäthanolamin in Betracht.

Die als Ausgangsstoffe verwendeten Verbindungen sind teilweise literaturbekannt oder man erhält diese nach literaturbekannten Verfahren wie diese in den Beispielen I bis X beschrieben werden.

Beispielsweise erhält man eine Verbindung der allgemeinen Formel II durch Umsetzung eines entsprechenden Nitrils mit einem Alkohol wie Methanol, Ethanol oder Propanol in Gegenwart einer Säure wie Salzsäure oder durch Umsetzung eines entsprechenden Amids mit einem Trialkyloxoniumsalz wie Triethyloxonium-tetrafluoroborat zweckmäßigerweise in einem Lösungsmittel wie Methylenchlorid, Tetrahydrofuran oder Dioxan oder durch Umsetzung eines entsprechenden Nitrils mit Schwefelwasserstoff zweckmäßigerweise in einem Lösungsmittel wie Pyridin oder Dimethylformamid und anschließende Alkylierung des so erhaltenen Thioamids mit einem Alkylhalogenid sowie anschließender Umsetzung eines so erhaltenen Iminoesters oder Iminothioesters mit Ammoniak, Methylamin oder mit einem entsprechenden Säureadditionssalz wie Ammoniumcarbonat oder Ammoniumacetat.

Beispielsweise erhält man eine Verbindung der allgemeinen Formel IV durch Spaltung eines entsprechenden Esters in einem wässrigen Lösungsmittel, z. B. in Wasser, Wasser/Tetrahydrofuran oder Wasser/Methanol in Gegenwart einer Alkalibase wie Natriumhydroxid oder Kaliumhydroxid oder in Gegenwart einer Säure wie Salzsäure, Schwefelsäure oder Trifluoressigsäure. Eine Verbindung der allgemeinen Formel IV, in der B eine durch eine Alkoxycarbonyl- oder Benzyloxycarbonylgruppe substituierte Amidinogruppe darstellt, erhält man beispielsweise dadurch, daß vor der vorstehend erwähnten Esterspaltung eine entsprechende Amidinoverbindung mit einem entsprechenden Alkoxycarbonyl- oder Benzyloxycarbonylhalogenid umgesetzt wird.

Wie bereits eingangs erwähnt, weisen die neuen 2-Pyrrolidinone der allgemeinen Formel I und deren Salze, insbesondere deren physiologisch verträgliche Salze mit anorganischen oder organischen Säuren oder Basen, wertvolle pharmakologische Eigenschaften auf, neben einer entzündungshemmenden und den Knochenabbau emmenden Wirkung insbesondere antithrombotische, antiaggregatorische und tumor- bzw. metastasenhemmende Wirkungen.

Beispielsweise wurden die Verbindungen der allgemeinen Formel I auf ihre biologischen Wirkungen wie folgt untersucht:

Die Hemmung der Thrombozytenaggregation nach oraler Gabe der Prüfsubstanz wird ex vivo an Rhesusaffen ermittelt.

Direkt vor der oralen Verabreichung der in Natrosol suspendierten Prüfsubstanz wird den Tieren eine Blutprobe aus der Kubitalvene als Referenzwert entnommen. Zu definierten Zeiten nach der Substanzgabe werden erneut Blutproben gewonnen und wie folgt untersucht.

Das mit 3,14% Natriumcitrat im Volumenverhältnis 1:10 versetzte Vollblut wird mit 200 g für 15 Minuten zentrifugiert. Der Überstand an plättchenreichem Plasma wird vorsichtig abgehoben. Aus dem erythrozytenreichen Sediment wird durch Zentrifugation mit 4000 g für 10 Minuten das plättchenarme Plasma als Überstand gewonnen.

Die mit Collagen (Hormonchemie, München; 2 mg/ml Endkonzentration im plättchenreichen Plasma) ausgelöste Thrombozytenaggregation in diesen ex vivo Proben wird nach der Methode von Born und Cross (J. Physiol. 170, 397 (1964)) photometrisch gemessen. Die nach Collagenstimulation gemessene größte Lichtdurchlässigkeit des plättchenreichen Plasmas wird mit dem Referenzwert verglichen, um die Hemmung der Aggregation zu den verschiedenen Zeitpunkten der Blutentnahme nach Substanzgabe relativ zum Referenzwert zu bestimmen.

Die Verbindungen der Beispiele 1 und 3 hemmen die Collageninduzierte Thrombozytenaggregation ex vivo nach oraler Gabe von 1 mg/kg länger als 8 bzw. länger als 4 Stunden.

Nach oraler Gabe von 3 mg/kg hemmt die Verbindung des Beispiels 4 die Collagen-induzierte Thrombozytenaggregation ex vivo länger als 8 Stunden.

Die erfindungsgemäßen Verbindungen sind gut verträglich, da beispielsweise die approximative $LD_{50}$ der Verbindungen der Beispiele 1 und 3 an der Maus oberhalb 300 mg/kg und die der Verbindung des Beispiels 4 oberhalb 100

mg/kg jeweils nach oraler Gabe liegt.

Auf Grund ihrer Hemmwirkung auf Zell-Zell- bzw. Zell-Matrix-Wechselwirkungen eignen sich die neuen 2-Pyrrolidinone der allgemeinen Formel I und ihre physiologisch verträglichen Salze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose und der Metastasierung von Tumoren und der Therapie genetisch bedingter oder auch erworbener Störungen der Interaktionen von Zellen untereinander oder mit soliden Strukturen. Weiterhin eignen sich die neuen Verbindungen zur Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 mg und 30 mg/kg Körpergewicht, vorzugsweise bei 1 mg bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromooxan-Rezeptor-Antagonisten und Thromboxansynthesehemmer oder deren Kombinationen, Serotonin-Antagonisten, a-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

Ausgangsverbindungen:

Beispiel I

(S)-1-(Benzyloxycarbonyl)-5-[(trityloxy)methyl]-2-pyrrolidinon

Eine Lösung von 160 g (S)-5-[(Trityloxy)methyl]-2-pyrrolidinon in 1600 ml trockenem Tetrahydrofuran wird innerhalb von 35 Minuten bei -65°C mit 179 ml einer 2,5 M Lösung von Butyllithium in Hexan versetzt. Nach 10 Minuten wird bei -65°C eine Lösung von 66,8 ml Chlorameisensäure-benzylester in 100 ml trockenem Tetrahydrofuran zugetropft und eine Stunde gerührt. Dann wird mit 200 ml gesättigter Kochsalzlösung versetzt und das Tetrahydrofuran abrotiert. Der Rückstand wird zwischen 3,5 l Essigester und 200 ml Wasser verteilt, die organische Phase abgetrennt und je zweimal mit Wasser und Kochsalzlösung gewaschen. Die organische Phase wird abgetrennt getrocknet und einrotiert. Das Rohprodukt wird aus wenig Ethanol umkristallisiert.

Ausbeute: 181 g (82 % der Theorie),
Schmelzpunkt: 103-105°C
$R_f$-Wert: 0,53 (Kieselgel; Cyclohexan/Essigester = 2:1)

| | | | |
|---|---|---|---|
| Ber.: | C 78,19 | H 5,95 | N 2,85 |
| Gef.: | 78,34 | 6,00 | 3,10 |

Beispiel II

(3S,5S)-1-(Benzyloxycarbonyl)-3-[(tert.butyloxycarbonyl)methyl]-5-[(trityloxy)methyl]-2-pyrrolidinon

Zu 20,0 g (S)-1-(Benzyloxycarbonyl)-5-[(trityloxy)methyl]-2-pyrrolidinon in 200 ml trockenem Tetrahydrofuran werden bei -65°C 40,6 ml einer 1 molaren Lösung von Lithiumnexamethyldisilazid in Tetrahydrofuran zugetropft. Nach 10 Minuten wird eine Lösung von 6,57 ml Bromessigsäure-tert.butylester in 20 ml trockenem Tetrahydrofuran zugetropft. Nach 2 Stunden Rühren bei -65°C wird auf 0°C erwärmt und 20 ml einer gesättigten wäßrigen Ammoniumchloridlösung zugegeben. Es wird im Vakuum eingeengt, der Rückstand mit 500 ml Essigester versetzt und zweimal mit Wasser und

einmal mit gesättigter Kochsalzlösung ausgeschüttelt. Die organische Phase wird getrocknet, einrotiert und der Rückstand durch Chromatographie über eine Kieselgelsäule mit Cyclohexan/Essigester = 4:1 gereinigt.

Ausbeute: 20,0 g (81 % der Theorie),
$R_f$-Wert: 0,66 (Kieselgel; Cyclohexan/Essigester = 2:1)

| | | | |
|---|---|---|---|
| Ber.: | C 75,35 | H 6,49 | N 2,31 |
| Gef.: | 75,17 | 6,65 | 2,50 |

Beispiel III

(3S,5S)-3-[(tert.Butyloxycarbonyl)methyl]-5-hydroxymethyl-2-pyrrolidinon

246 g (3S,5S)-1-(Benzyloxycarbonyl)-3-[(tert.butyloxycarbonyl)methyl]-5-[(trityloxy)methyl]-2-pyrrolidinon in 1,6 l tert.Butanol werden 1 1/2 Tage bei 50°C unter einem Wasserstoffdruck von 5 bar mit 50 g Palladium (10 % auf Aktivkohle) hydriert. Anschließend wird mit Aceton verdünnt, der Katalysator abfiltriert und das Filtrat eingeengt. Der Rückstand wird in 3 Portionen mit insgesamt 2 l Petrolether ausgerührt. Das verbleibende Öl wird im Vakuum getrocknet.

Ausbeute: 77,8 g (84 % der Theorie),
$R_f$-Wert: 0,43 (Kieselgel; Essigester/Methanol = 15:1)

Beispiel IV

(3S,5S)-3-[(tert.Butyloxycarbonyl)methyl]-5-[(methansulfonyloxy)methyl]-2-pyrrolidinon

Zu 2,7 g (3S,5S)-3-[(tert.Butyloxycarbonyl)methyl]-5-hydroxymethyl-2-pyrrolidinon in 25 ml Methylenchlorid werden bei 0°C 1,76 g Methansulfonsäurechlorid gegeben und dann 1,7 g Triethylamin zugetropft. Nach einer Stunde Rühren unter Eiskühlung und 2 Stunden Rühren bei Raumtemperatur wird mit Eiswasser versetzt, die organische Phase abgetrennt, zweimal mit Wasser gewaschen, getrocknet und eingeengt. Nach Umkristallisation aus Essigester werden 2,35 g (65 % der Theorie) erhalten.

Schmelzpunkt: 114-116°C
$R_f$-Wert: 0,47 (Kieselgel; Toluol/Aceton = 1:3)

| | | | | |
|---|---|---|---|---|
| Ber.: | C 46,89 | H 6,89 | N 4,56 | S 10,43 |
| Gef.: | 46,90 | 6,79 | 4,84 | 10,17 |

Beispiel V

(3S,5S)-3-[(tert.Butyloxycarbonyl)methyl]-5-[(4'-cyano-4-biphenylyl)oxymethyl]-2-pyrrolidinon

Zu 5,9 g 4'-Cyano-4-hydroxybiphenyl in 150 ml trockenem Dimethylformamid werden 3,4 g Kalium-tert.butylat gegeben. Nach einer Stunde Rühren bei Raumtemperatur werden 9,2 g (3S,5S)-3-[(tert.Butyloxycarbonyl)methyl]-5-[(methansulfonyloxy)methyl]-2-pyrrolidinon zugegeben und 3 Tage bei Raumtemperatur gerührt. Das Reaktionsgemisch wird auf 550 ml Wasser gegeben und das Gemisch eine halbe Stunde gerührt. Der Niederschlag wird abgesaugt und zweimal aus 95%igem Ethanol umkristallisiert.

Ausbeute: 8,35 g (68 % der Theorie),
Schmelzpunkt: 159-160°C
$R_f$-Wert: 0,64 (Kieselgel; Essigester)

Beispiel VI

(3S,5S)-5-[(4'-Amidino-4-biphenylyl)oxymethyl]-3-[(methoxycarbonyl)methyl]-2-pyrrolidinon-hydrochlorid

0,9 g (3S,5S)-3-[(tert.Butyloxycarbonyl)methyl]-5-[(4'-cyano-4-biphenylyl)oxymethyl]-2-pyrrolidinon werden zu 50 ml trockenem Methanol, das unter Eiskühlung mit Salzsäuregas gesättigt wurde, gegeben. Das Reaktionsgemisch wird mit etwas Petrolether überschichtet und über Nacht gerührt. Es wird eingeengt, der Rückstand mit 11 l Methanol versetzt und unter Eiskühlung mit konz. wäßrigem Ammoniak ein pH-Wert von ca. 9 eingestellt. Nach 3 Tagen Rühren bei Raumtemperatur wird eingeengt, der Rückstand mit 100 ml Methylenchlorid/Methanol (85:15) gerührt, dann wird filtriert und das Filtrat eingeengt. Der Rückstand wird mit Methylenchlorid/tert.Butylmethylether verrührt und der Feststoff abgesaugt und getrocknet.

Ausbeute: 0,8 g (87 % der Theorie)
$R_f$-Wert: 0,45 (Reversed Phase Kieselgel (RP8); Methanol/5%ige wäßrige Kochsalzlösung = 3:2)

Beispiel VII

(3S,5S)-5-[(4'-Methoxycarbonylamidino-4-biphenylyl)oxymethyl]-3-[(methoxycarbonyl)methyl]-2-pyrrolidinon

Zu einer Suspension von 2,0 g (3S,5S)-5-[(4'-Amidino-4-biphenylyl)oxymethyl]-3-[(methoxycarbonyl)methyl]-2-pyrrolidinon-hydrochlorid in 200 ml Methylenchlorid werden 480 mg Chlorameisensäure-methylester zugegeben und dann unter starkem Rühren bei Raumtemperatur 50 ml 0,2 molarer Natronlauge zugetropft. Die organische Phase wird abgetrennt, zweimal mit Wasser gewaschen, getrocknet und eingeengt. Der Rückstand wird über eine Kieselgelsäule mit Essigester und Essigester/Methanol (95:5) chromatographiert. Das Produkt wird mit 15 ml Essigester zum Sieden erhitzt, die Suspension abgekühlt und der Feststoff abgesaugt und getrocknet.

Ausbeute: 430 mg (21 % der Theorie),
Schmelzpunkt: 183-184°C (Zers.)
$R_f$-Wert: 0,47 (Kieselgel; Essigester/Methanol = 97:3)

| | | | |
|---|---|---|---|
| Ber.: | C 62,86 | H 5,73 | N 9,56 |
| Gef.: | 62,60 | 5,85 | 9,55 |

Analog wird erhalten:

(1) (3S,5S)-5-[(4'-Benzyloxycarbonylamidino-4-biphenylyl)oxymethyl]-3-[(methoxycarbonyl)methyl]-2-pyrrolidinon (Durchführung mit Chlorameisensäurebenzylester in Tetrahydrofuran)

$R_f$-Wert: 0,37 (Kieselgel; Essigester)

Beispiel VIII

(3S,5S)-3-Carboxymethyl-5-[(4'-methoxycarbonylamidino-4-biphenylyl)oxymethyl]-2-pyrrolidinon

440 mg (3S,5S)-5-[(4'-Methoxycarbonylamidino-4-biphenylyl)oxymethyl]-3-[(methoxycarbonyl)methyl]-2-pyrrolidinon werden in einer Mischung aus 8 ml Tetrahydrofuran und 4 ml Wasser suspendiert und bei Raumtemperatur unter Rühren mit 0,75 ml 4N Natronlauge versetzt. Nach 30 Minuten Rühren bei Raumtemperatur wird das Reaktionsgemisch mit Eisessig angesäuert und mit etwas Wasser verdünnt. Der Niederschlag wird abgesaugt, mit Wasser gewaschen und getrocknet.

Ausbeute: 320 mg (75 % der Theorie)
$R_f$-Wert: 0,68 (Reversed Phase Kieselgel (RP8); Methanol/5%ige wäßrige Kochsalzlösung = 6:4)

Analog wird erhalten:

(1) (3S,5S)-5-[(4'-Benzyloxycarbonylamidino-4-biphenylyl)oxymethyl]-3-carboxymethyl-2-pyrrolidinon

Schmelzpunkt: 202°C (Zers.)
$R_f$-Wert: 0,44 (Reversed Phase Kieselgel (RP8); Methanol/5%ige wäßrige Kochsalzlösung = 6:4)

Beispiel IX

(3S,5S)-5-[(4'-Amidino-4-biphenylyl)oxymethyl]-3-carboxymethyl-2-pyrrolidinon

Zu 20 g (3S,5S)-5-[(4'-Amidino-4-biphenylyl)oxymethyl]-3-[(methoxycarbonyl)methyl]-2-pyrrolidinon-hydrochlorid in 200 ml Methanol werden 37,5 ml 4N Natronlauge gegeben und 6 Stunden bei Raumtemperatur gerührt. Es werden 10,7 g Ammoniumchlorid und nach einigen Minuten noch 200 ml Wasser zugegeben und 45 Minuten in einem Eis/Wasser-Bad gerührt. Der Niederschlag wird abgesaugt, je zweimal mit Methanol und Wasser/Methanol (2:1) gewaschen und getrocknet.

Ausbeute: 15,2 g (86 % der Theorie),
Schmelzpunkt: 292-294°C (Zers.)
$R_f$-Wert: 0,63 (Reversed Phase Kieselgel (RP8); Methanol/10%ige wäßrige Kochsalzlösung = 6:4)

| | | | |
|---|---|---|---|
| Ber.: | C 65,38 | H 5,76 | N 11,44 |
| Gef.: | 65,20 | 5,80 | 11,54 |

Beispiel X

(3S,5S)-5-[(4'-Amidino-4-biphenylyl)oxymethyl]-3-[(neopentyloxycarbonyl)methyl]-2-pyrrolidinon x 1,1 HCl x 0,5 Wasser

Über eine Suspension von 1,1 g (3S,5S)-5-[(4'-Amidino-4-biphenylyl)oxymethyl]-3-carboxymethyl-2-pyrroldinon in 10 g flüssigem Neopentylalkohol wird unter Rühren Salzsäuregas geleitet. Nach 20 Minuten wird das Reaktionsgemisch noch eine Stunde bei 60°C gerührt. Das Reaktionsgemisch wird nach dem Abkühlen mit Diethylether versetzt und der Niederschlag abgesaugt und im Exsikkator über Kaliumhydroxid und konz. Schwefelsäure getrocknet. Das Rohprodukt wird unter leichtem Erwärmen in Methylenchlorid aufgenommen, filtriert und das Filtrat eingeengt. Der Rückstand wird mit Aceton/Diethylether verrührt, abgesaugt, mit Diethylether gewaschen und über Natriumhydroxid und konz. Schwefelsäure im Exsikkator getrocknet.

Ausbeute: 1,14 g (80 % der Theorie),
$R_f$-Wert: 0,24 (Reversed Phase Kieselgel (RP8); Methanol/5%ige wäßrige Kochsalzlösung = 6:4)

| | | | | |
|---|---|---|---|---|
| Ber.: | C 61,70 | H 6,86 | N 8,63 | Cl 8,01 |
| Gef.: | 61,98 | 7,14 | 8,43 | 7,87 |

Massenspektrum: $(M+H)^+$ = 438

Analog werden erhalten:

(1) (3S,5S)-5-[(4'-Amidino-4-biphenylyl)oxymethyl]-3-[(isobutyloxycarbonyl)methyl]-2-pyrrolidinon-hydrochlorid

$R_f$-Wert: 0,26 (Reversed Phase Kieselgel (RP8); Methanol/5%ige wäßrige Kochsalzlösung = 6:4)
Massenspektrum: $(M+H)^+$ = 424

(2) (3S,5S)-5-[(4'-Amidino-4-biphenylyl)oxymethyl]-3-[(benzyloxycarbonyl)methyl]-2-pyrrolidinon x 1,05 HCl x 0,5 Wasser

$R_f$-Wert: 0,19 (Reversed Phase Kieselgel (RP8); Methanol/5%ige wäßrige Kochsalzlösung = 6:4)

| | | | | |
|---|---|---|---|---|
| Ber.: | C 64,24 | H 5,80 | N 8,32 | Cl 7,37 |
| Gef.: | 64,38 | 5,73 | 8,20 | 7,42 |

Massenspektrum: $(M+H)^+$ = 458

(3) (3S,5S)-5-[(4'-Amidino-4-biphenylyl)oxymethyl]-3-[(ethyloxycarbonyl)methyl]-2-pyrrolidinon-hydrochlorid

$R_f$-Wert: 0,54 (Reversed Phase Kieselgel (RP8); Methanol/10%ige wäßrige Kochsalzlösung = 6:4)

(4) (3S,5S)-5-[(4'-Amidino-4-biphenylyl)oxymethyl]-3-[(isopropyloxycarbonyl)methyl]-2-pyrrolidinon-hydrochlorid

$R_f$-Wert: 0,49 (Reversed Phase Kieselgel (RP8); Methanol/10%ige wäßrige Kochsalzlösung = 6:4)

(5) (3S,5S)-5-[(4'-Amidino-4-biphenylyl)oxymethyl]-3-[(propyloxycarbonyl)methyl]-2-pyrrolidinon-hydrochlorid

## Beispiel 1

(3S,5S)-5-[(4'-Acetoxymethyloxycarbonylamidino-4-biphenylyl)-oxymethyl]-3-[(methoxycarbonyl)methyl]-2-pyrrolidinon

1,06 g (3S,5S)-5-[(4'-Amidino-4-biphenylyl)oxymethyl]-3-[(methoxycarbonyl)methyl]-2-pyrrolidinon-hydrochlorid, 800 mg Kohlensäure-acetoxymethyl-(4-nitrophenyl)-ester und 726 mg N-Ethyldiisopropylamin werden in 80 ml Methylenchlorid 20 Stunden bei Raumtemperatur gerührt. Das Reaktionsgemisch wird mit Wasser, mit 40 ml 0,1N Natronlauge und wieder mit Wasser gewaschen. Anschließend wird die organische Phase abgetrennt, getrocknet und eingeengt. Der Rückstand wird über eine Kieselgelsäule mit Methylenchlorid/Methanol (97:3) chromatographiert. Das Produkt wird in der Wärme kurz mit tert.Butyl-methylether verrührt, dann abgesaugt und getrocknet.

Ausbeute: 380 mg (30 % der Theorie),
$R_f$-Wert: 0,25 (Kieselgel; Methylenchlorid/Methanol = 95:5)

| | | | |
|---|---|---|---|
| Ber.: | C 60,36 | H 5,47 | N 8,45 |
| Gef.: | 60,33 | 5,87 | 8,07 |

Massenspektrum: $(M+H)^+$ = 498

Analog werden erhalten:

(1) (3S,5S)-5-[[4'-(1-Acetoxyethyl)oxycarbonylamidino-4-biphenylyl]oxymethyl]-3-[(methoxycarbonyl)methyl]-2-pyrrolidinon x 0,75 Wasser

$R_f$-Wert: 0,36 (Kieselgel; Methylenchlorid/Aceton = 65:35)

| | | | |
|---|---|---|---|
| Ber.: | C 59,48 | H 5,86 | N 8,00 |
| Gef.: | 59,38 | 5,73 | 8,02 |

Massenspektrum: $(M+H)^+$ = 512

(2) (3S,5S)-5-[[4'-(1-Acetoxyethyl)oxycarbonylamidino-4-biphenylyl]oxymethyl]-3-[(ethoxycarbonyl)methyl]-2-pyrrolidinon

(3) (3S,5S)-5-[(4'-Acetoxymethyloxycarbonylamidino-4-biphenylyl)oxymethyl]-3-[(ethoxycarbonyl)methyl]-2-pyrrolidinon

(4) (3S,5S)-5-[(4'-Acetoxymethyloxycarbonylamidino-4-biphenylyl)oxymethyl]-3-[(isopropoxycarbonyl)methyl]-2-pyrrolidinon

(5) (3S,5S)-5-[(4'-Acetoxymethyloxycarbonylamidino-4-biphenylyl)oxymethyl]-3-[(benzyloxycarbonyl)methyl]-2-pyrrolidinon

(6) (3S,5S)-3-[(Methoxycarbonyl)methyl]-5-[(4'-pivaloyloxymethyloxycarbonylamidino-4-biphenylyl)oxymethyl]-2-pyrrolidinon

(7) (3S,5S)-3-[(Ethoxycarbonyl)methyl]-5-[(4'-pivaloyloxymethyloxycarbonylamidino-4-biphenylyl)oxymethyl]-2-pyrrolidinon

(8) (3S,5S)-5-[(4'-Butyryloxymethyloxycarbonylamidino-4-biphenylyl)oxymethyl]-3-[(methoxycarbonyl)methyl]-2-pyrrolidinon

(9) (3S,5S)-5-[(4'-Acetoxymethyloxycarbonylamidino-4-biphenylyl)oxymethyl]-3-[(propoxycarbonyl)methyl]-2-pyrrolidinon

(10) (3S,5S)-5-[(4'-Acetoxymethyloxycarbonylamidino-4-biphenylyl)oxymethyl]-3-[(pivaloyloxymethyloxycarbonyl)methyl]-2-pyrrolidinon

(11) (3S,5S)-5-[(4'-Acetoxymethyloxycarbonylamidino-4-biphenylyl)sulfonylmethyl]-3-[(methoxycarbonyl)methyl]-1-(3-phenylpropyl)-2-pyrrolidinon

(12) (3S,5S)-3-[(Cyclohexyloxycarbonyl)methyl]-5-[(4'-propionyloxymethyloxycarbonylamidino-4-biphenylyl)oxymethyl]-2-pyrrolidinon

(13) (3S,5S)-5-[(4'-Isobutyryloxymethyloxycarbonylamidino-4-biphenylyl)oxymethyl]-3-[(methoxycarbonyl)methyl]-2-pyrrolidinon

(14) (3S,5S)-5-[(4'-Acetoxymethyloxycarbonylamidino-4-biphenylyl)oxymethyl]-3-[(neopentyloxycarbonyl)methyl]-2-pyrrolidinon

(15) (3S,5S)-5-[(4'-Acetoxymethyloxycarbonylamidino-4-biphenylyl)oxymethyl]-3-[(isobutyloxycarbonyl)methyl]-2-pyrrolidinon

Beispiel 2

(3S,5S)-5-[(4'-Benzyloxycarbonylamidino-4-biphenylyl)oxymethyl]-3-[[[1-(cyclohexyloxycarbonyloxy)ethyl]oxycarbonyl]methyl]-2-pyrrolidinon

900 mg (3S,5S)-5-[(4'-Benzyloxycarbonylamidino-4-biphenylyl)oxymethyl]-3-carboxymethyl-2-pyrrolidinon, 750 mg Kohlensäure-(1-chlorethyl)-cyclohexylester, 500 mg Kaliumcarbonat und eine Spatelspitze Natriumjodid werden in 8 ml Dimethylsulfoxid 3 Tage bei Raumtemperatur gerührt. Es wird mit Eiswasser versetzt und der Niederschlag abgesaugt und mit Wasser gewaschen. Der feuchte Niederschlag wird in Essigester aufgenommen, mit Magnesiumsulfat getrocknet, filtriert und einrotiert. Der Rückstand wird mit Diethylether gerührt, das Festprodukt wird abgesaugt mit Diethylether gewaschen und getrocknet.

Ausbeute: 1,5 g (95 % der Theorie),
$R_f$-Wert: 0,60 (Kieselgel; Methylenchlorid/Aceton = 65:35)

Analog werden erhalten:

(1) (3S,5S)-5-[(4'-Benzyloxycarbonylamidino-4-biphenylyl)oxymethyl]-3-[[[1-(ethyloxycarbonyloxy)ethyl]oxycarbonyl]methyl]-2-pyrrolidinon

$R_f$-Wert: 0,43 (Kieselgel; Methylenchlorid/Aceton = 65:35)

(2) (3S,5S)-5-[(4'-Benzyloxycarbonylamidino-4-biphenylyl)oxymethyl]-3-[(pivaloyloxymethyloxycarbonyl)methyl]-2-pyrrolidinon

$R_f$-Wert: 0,52 (Kieselgel; Methylenchlorid/Aceton = 65:35)

(3) (3S,5S)-3-[(Acetoxymethyloxycarbonyl)methyl]-5-[(4'-benzyloxycarbonylamidino-4-biphenylyl)oxymethyl]-2-pyrrolidinon

(4) (3S,5S)-5-[(4'-Benzyloxycarbonylamidino-4-biphenylyl)oxymethyl]-3-[(butyryloxymethyloxycarbonyl)methyl]-2-pyrrolidinon

(5) (3S,5S)-5-[(4'-Benzyloxycarbonylamidino-4-biphenylyl)oxymethyl]-3-[(isobutyryloxymethyloxycarbonyl)methyl]-2-pyrrolidinon

(6) (3S,5S)-3-[[(1-Acetoxyethyl)oxycarbonyl]methyl]-5-[(4'-benzyloxycarbonylamidino-4-biphenylyl)oxymethyl]-2-pyrrolidinon

(7) (3S,5S)-3-[(Benzoyloxymethyloxycarbonyl)methyl]-5-[(4'-benzyloxycarbonylamidino-4-biphenylyl)oxymethyl]-2-pyrrolidinon

(8) (3S,5S)-5-[(4'-Benzyloxycarbonylamidino-4-biphenylyl)oxymethyl]-3-[[(cyclohexyloxycarbonyloxy)methyloxycarbonyl]methyl]-2-pyrrolidinon

(9) (3S,5S)-5-[(4'-Benzyloxycarbonylamidino-4-biphenylyl)oxymethyl]-3-[[(cyclopentyloxycarbonyloxy)methyloxycarbonyl]methyl]-2-pyrrolidinon

(10) (3S,5S)-5-[(4'-Benzyloxycarbonylamidino-4-biphenylyl)oxymethyl]-1-phenyl-3-[(pivaloyloxymethyloxycarbonyl)methyl]-2-pyrrolidinon

(11) (3S,5S)-1-Benzyl-5-[(4'-benzyloxycarbonylamidino-4-biphenylyl)oxymethyl]-3-[(pivaloyloxymethyloxycarbonyl)methyl]-2-pyrrolidinon

(12) (3S,5S)-5-[(4'-Benzyloxycarbonylamidino-4-biphenylyl)oxymethyl]-1-[2-(3,4-dimethoxyphenyl)ethyl]-3-[(pivaloyloxymethyloxycarbonyl)methyl]-2-pyrrolidinon

(13) (3S,5S)-5-[(4'-Benzyloxycarbonylamidino-4-biphenylyl)oxymethyl]-1-(2-methoxyethyl)-3-[(pivaloyloxymethyloxycarbonyl)methyl]-2-pyrrolidinon

(14) (3S,5S)-5-[(4'-Benzyloxycarbonylamidino-4-biphenylyl)oxymethyl]-1-[(morpholino-N-carbonyl)methyl]-3-[(pivaloyloxymethyloxycarbonyl)methyl]-2-pyrrolidinon

(15) (3S,5S)-5-[(4'-Benzyloxycarbonylamidino-4-biphenylyl)oxymethyl]-3-[[[1-(pivaloyloxy)butyl]oxycarbonyl]methyl]-2-pyrrolidinon

(16) (3S,5S)-5-[(4'-Benzyloxycarbonylamidino-4-biphenylyl)oxymethyl]-3-[[[1-(butyloxycarbonyloxy)ethyl]oxycarbonyl]methyl]-2-pyrrolidinon

(17) (3S,5S)-5-[(4'-Benzyloxycarbonylamidino-4-biphenylyl)oxymethyl]-3-[[(cyclohexylcarbonyloxymethyl)oxycarbonyl]methyl]-2-pyrrolidinon

(18) (3R,S;4R,S)-4-[(4'-Benzyloxycarbonylamidino-4-biphenylyl)oxymethyl]-3-methyl-3-[(pivaloyloxymethyloxycarbonyl)methyl]-2-pyrrolidinon

(19) (3R,S;4R,S)-4-[(4'-Benzyloxycarbonylamidino-4-biphenylyl)carbonylaminomethyl]-3-[[[1-(ethyloxycarbonyloxy)ethyl]oxycarbonyl[methyl]-2-pyrrolidinon

(20) (3S,5S)-5-[(4'-Benzyloxycarbonylamidino-3-fluor-4-biphenylyl)oxymethyl]-3-[(pivaloyloxymethyloxycarbonyl)methyl]-2-pyrrolidinon

(21) (3S,5S)-5-[(7-Benzyloxycarbonylamidino-9,10-dihydro-2-phenanthrenyl)oxymethyl]-3-[[[1-(cyclohexyloxycarbonyloxy)ethyl]oxycarbonyl]methyl]-2-pyrrolidinon

(22) (3S,5S)-5-[(7-Benzyloxycarbonylamidino-2-fluorenyl)oxymethyl]-3-[(pivaloyloxymethyloxycarbonyl)methyl]-2-pyrrolidinon

(23) (3S,5S)-5-[(4'-Methoxycarbonylamidino-4-biphenylyl)oxymethyl]-3-[(pivaloyloxymethyloxycarbonyl)methyl]-2-pyrrolidinon x 0,5 Wasser

$R_f$-Wert: 0,35 (Kieselgel; Methylenchlorid/Aceton = 65:35)

| | | | |
|---|---|---|---|
| Ber.: | C 61,30 | H 6,25 | N 7,66 |
| Gef.: | 61,40 | 6,20 | 7,38 |

Massenspektrum: $(M+H)^+$ = 540

(24) (3S,5S)-3-[[[1-(Ethyloxycarbonyloxy)ethyl]oxycarbonyl]methyl]-5-[(4'-methoxycarbonylamidino-4-biphenylyl)oxymethyl]-2-pyrrolidinon

(25) (3S,5S)-3-[[[1-(Cyclohexyloxycarbonyloxy)ethyl]oxycarbonyl]methyl]-5-[(4'-methoxycarbonylamidino-4-biphenylyl)oxymethyl]-2-pyrrolidinon

(26) (3S,5S)-5-[(4'-Ethoxycarbonylamidino-4-biphenylyl)oxymethyl]-3-[(pivaloyloxymethyloxycarbonyl)methyl]-2-pyrrolidinon

(27) (3S,5S)-5-[(4'-Benzyloxycarbonylamidino-4-biphenylyl)oxymethyl]-3-[[[1-(propionyloxy)ethyl]oxycarbonyl]methyl]-2-pyrrolidinon

(28) (3S,5S)-5-[(4'-Benzyloxycarbonylamidino-4-biphenylyl)oxymethyl]-3-[[[1-(cycloheptyloxycarbonyloxy)ethyl]oxycarbonyl]methyl]-2-pyrrolidinon

(29) (3S,5S)-5-[(4'-Benzyloxycarbonylamidino-4-biphenylyl)oxymethyl]-3-[[[1-(isopropyloxycarbonyloxy)ethyl]oxycarbonyl]methyl]-2-pyrrolidinon

(30) (3S,5S)-5-[(4'-Benzyloxycarbonylamidino-4-biphenylyl)oxymethyl]-3-[[[1-(isobutyloxycarbonyloxy)ethyl]oxycarbonyl]methyl]-2-pyrrolidinon

(31) (3S,5S)-5-[(4'-Benzyloxycarbonylamidino-4-biphenylyl)oxymethyl]-3-[[[1-(tert.butyloxycarbonyloxy)ethyl]oxycarbonyl]methyl]-2-pyrrolidinon

(32) (3S,5S)-5-[(4'-Benzyloxycarbonylamidino-4-biphenylyl)oxymethyl]-3-[[[1-(propyloxycarbonyloxy)ethyl]oxycarbonyl]methyl]-2-pyrrolidinon

Beispiel 3

(3S,5S)-5-[(4'-Amidino-4-biphenylyl)oxymethyl]-3-[[[1-(cyclohexyloxycarbonyloxy)ethyl]oxycarbonyl]methyl]-2-pyrrolidinon x 1,1 HCl x 0,5 Wasser

1,1 g (3S,5S)-5-[(4'-Benzyloxycarbonylamidino-4-biphenylyl]oxymethyl]-3-[[[1-(cyclohexyloxycarbonyloxy)ethyl]oxycarbonyl]methyl]-2-pyrrolidinon, gelöst in 10 ml Dimethylformamid, werden mit 3,26 ml 0,5N Salzsäure versetzt und mit 0,2 g Palladium auf Kohle (10 % Palladium) bei Raumtemperatur und einem Wasserstoffdruck von 3,4 bar hydriert. Nach 2 1/2 Stunden wird abgesaugt, das Filtrat mit gesättigter Kochsalzlösung versetzt und dreimal mit Tetrahydrofuran ausgeschüttelt. Die vereinigten organischen Phasen werden mit gesättigter Kochsalzlösung gewaschen, filtriert und eingeengt. Der Rückstand wird mit Diethylether verrührt und die überstehende Lösung abdekantiert. Der Rückstand wird im Exsikkator über konzentrierter Schwefelsäure und Kaliumhydroxid getrocknet. Dann wird mit Diethylether verrieben, abgesaugt mit Diethylether gewaschen und getrocknet.

Ausbeute: 700 mg (75 % der Theorie),
$R_f$-Wert: 0,16 (Reversed Phase Kieselgel (RP8); Methanol/5%ige wäßrige Kochsalzlösung = 6:4)

| | | | | |
|---|---|---|---|---|
| Ber.: | C 59,37 | H 6,37 | N 7,16 | Cl 6,65 |
| Gef.: | 58,94 | 6,45 | 7,14 | 6,77 |

Massenspektrum: $(M+H)^+$ = 538

Analog werden erhalten:

(1) (3S,5S)-5-[(4'-Amidino-4-biphenylyl)oxymethyl]-3-[[[1-(ethyloxycarbonyloxy)ethyl]oxycarbonyl]methyl]-2-pyrrolidinon x 1,05 HCl x 0,5 Wasser

$R_f$-Wert: 0,34 (Reversed Phase Kieselgel (RP8); Methanol/5 %ige wäßrige Kochsalzlösung = 6:4)

| | | | | |
|---|---|---|---|---|
| Ber.: | C 56,57 | H 5,90 | N 7,92 | Cl 7,01 |
| Gef.: | 56,51 | 5,93 | 7,88 | 7,10 |

Massenspektrum: $(M+H)^+$ = 484

(2) (3S,5S)-5-[(4'-Amidino-4-biphenylyl)oxymethyl]-3-[(pivaloyloxymethyloxycarbonyl)methyl]-2-pyrrolidinon x 1,1 HCl x 0,5 Wasser

$R_f$-Wert: 0,30 (Reversed Phase Kieselgel (RP8); Methanol/5 %ige wäßrige Kochsalzlösung = 6:4)

| | | | | |
|---|---|---|---|---|
| Ber.: | C 58,85 | H 6,29 | N 7,92 | Cl 7,35 |
| Gef.: | 58,69 | 6,21 | 7,75 | 7,40 |

Massenspektrum: $(M+H)^+$ = 482

(3) (3S,5S)-3-[(Acetoxymethyloxycarbonyl)methyl]-5-[(4'-amidino-4-biphenylyl)oxymethyl]-2-pyrrolidinon-hydrochlorid

(4) (3S,5S)-5-[(4'-Amidino-4-biphenylyl)oxymethyl]-3-[(butyryloxymethyloxycarbonyl)methyl]-2-pyrrolidinon-hydrochlorid

(5) (3S,5S)-5-[(4'-Amidino-4-biphenylyl)oxymethyl]-3-[(isobutyryloxymethyloxycarbonyl)methyl]-2-pyrrolidinon-hydrochlorid

(6) (3S,5S)-3-[[(1-Acetoxyethyl)oxycarbonyl]methyl]-5-[(4'-amidino-4-biphenylyl)oxymethyl]-2-pyrrolidinon-hydrochlorid

(7) (3S,5S)-5-[(4'-Amidino-4-biphenylyl)oxymethyl]-3-[(benzoyloxymethyloxycarbonyl)methyl]-2-pyrrolidinon-hydrochlorid

(8) (3S,5S)-5-[(4'-Amidino-4-biphenylyl)oxymethyl]-3-[[(cyclohexyloxycarbonyloxy)methyloxycarbonyl]methyl]-2-pyrrolidinon-hydrochlorid

(9) (3S,5S)-5-[(4'-Amidino-4-biphenylyl)oxymethyl]-3-[[(cyclopentyloxycarbonyloxy)methyloxycarbonyl]methyl]-2-pyrrolidinon-hydrochlorid

(10) (3S,5S)-5-[(4'-Amidino-4-biphenylyl)oxymethyl]-1-phenyl-3-[(pivaloyloxymethyloxycarbonyl)methyl]-2-pyrrolidinon-hydrochlorid

(11) (3S,5S)-5-[(4'-Amidino-4-biphenylyl)oxymethyl]-1-benzyl-3-[(pivaloyloxymethyloxycarbonyl)methyl]-2-pyrrolidinon-hydrochlorid

(12) (3S,5S)-5-[(4'-Amidino-4-biphenylyl)oxymethyl]-1-[2-(3,4-dimethoxyphenyl)ethyl]-3-[(pivaloyloxymethyloxycarbonyl)methyl]-2-pyrrolidinon-hydrochlorid

(13) (3S,5S)-5-[(4'-Amidino-4-biphenylyl)oxymethyl]-1-(2-methoxyethyl)-3-[(pivaloyloxymethyloxycarbonyl)methyl]-2-pyrrolidinon-hydrochlorid

(14) (3S,5S)-5-[(4'-Amidino-4-biphenylyl)oxymethyl]-1-[(morpholino-N-carbonyl)methyl]-3-[(pivaloyloxymethyloxycarbonyl)methyl]-2-pyrrolidinon-hydrochlorid

(15) 3S,5S)-5-[(4'-Amidino-4-biphenylyl)oxymethyl]-3-[[1-pivaloyloxy)butyl]oxycarbonylmethyl]-2-pyrrolidinon-hydrochlorid

(16) (3S,5S)-5-[(4'-Amidino-4-biphenylyl)oxymethyl]-3-[[[1-butyloxycarbonyloxy)ethyl]oxycarbonyl]methyl-2-pyrrolidinon-hydrochlorid

(17) (3S,5S)-5-[(4'-Amidino-4-biphenylyl)oxymethyl]-3-[[(cyclohexylcarbonyloxymethyl)oxycarbonyl]methyl]-2-pyrrolidinon-hydrochlorid

(18) (3R,S;4R,S)-4-[(4'-Amidino-4-biphenylyl)oxymethyl]-3-methyl-3-[(pivaloyloxymethyloxycarbonyl)methyl]-2-pyrrolidinon-hydrochlorid

(19) (3R,S;4R,S)-4-[(4'-Amidino-4-biphenylyl)carbonylaminomethyl]-3-[[[1-(ethyloxycarbonyloxy)ethyl]oxycarbonyl]methyl]-2-pyrrolidinon-hydrochlorid

(20) (3S,5S)-5-[(4'-Amidino-3-fluor-4-biphenylyl)oxymethyl]-3-[(pivaloyloxymethyloxycarbonyl)methyl]-2-pyrrolidinon-hydrochlorid

(21) (3S,5S)-5-[(7-Amidino-9,10-dihydro-2-phenanthrenyl)oxymethyl]-3-[[[1-(cyclohexyloxycarbonyloxy)ethyl]oxycarbonyl]methyl]-2-pyrrolidinon-hydrochlorid

(22) (3S,5S)-5-[(7-Amidino-2-fluorenyl)oxymethyl]-3-[(pivaloyloxymethyloxycarbonyl)methyl]-2-pyrrolidinon-hydrochlorid

(23) (3S,5S)-5-[(4'-Amidino-4-biphenyly1)oxymethyl]-3-[[[1-(propionyloxy)ethyl]oxycarbonyl]methyl]-2-pyrrolidinon-hydrochlorid

(24) (3S,5S)-5-[(4'-Amidino-4-biphenylyl)oxymethyl]-3-[[[1-(cycloheptyloxycarbonyloxy)ethyl]oxycarbonyl]methyl]-2-pyrrolidinon-hydrochlorid

(25) (3S,5S)-5-[(4'-Amidino-4-biphenylyl)oxymethyl]-3-[[[1-(isopropyloxycarbonyloxy)ethyl]oxycarbonyl]methyl]-2-pyrrolidinon-hydrochlorid

(26) (3S,5S)-5-[(4'-Amidino-4-biphenylyl)oxymethyl]-3-[[[1-(isobutyloxycarbonyloxy)ethyl]oxycarbonyl]methyl]-2-pyrrolidinon-hydrochlorid

(27) (3S,5S)-5-[(4'-Amidino-4-biphenylyl)oxymethyl]-3-[[[1-(tert.butyloxycarbonyloxy)ethyl]oxycarbonyl]methyl]-2-pyrrolidinon-hydrochlorid

(28) (3S,5S)-5-[(4'-Amidino-4-biphenylyl)oxymethyl]-3-[[[1-(propyloxycarbonyloxy)ethyl]oxycarbonyl]methyl]-2-pyrrolidinon-hydrochlorid

Beispiel 4

(3S,5S)-5-[(4'-Amidino-4-biphenylyl)oxymethyl]-3-[(cyclohexyloxycarbonyl)methyl]-2-pyrrolidinon x 1,05 HCl x 0,5 Wasser

Über eine Suspension von 1,5 g (3S,5S)-5-[(4'-Amidino-4-biphenylyl)oxymethyl]-3-carboxymethyl-2-pyrrolidinon in 15 ml Cyclohexanol wird unter Rühren Salzsäuregas geleitet. Das Reaktionsgemisch wird dann 2 Stunden bei 70°C gerührt, während dessen ab und zu Salzsäuregas darübergeleitet wird. Das Reaktionsgemisch wird abgekühlt, mit Ether versetzt und der Niederschlag abgesaugt und im Exsikkator über Kaliumhydroxid und konz. Schwefelsäure getrocknet. Das Rohprodukt wird in 400 ml Methylenchlorid aufgenommen, filtriert und eingeengt. Der Rückstand wird mit Aceton/Diethylether verrührt, abgesaugt, mit Diethylether gewaschen und im Exsikkator und im Trockenschrank bei 40°C getrocknet.

Ausbeute: 1,7 g (85 % der Theorie),
$R_f$-Wert: 0,26 (Reversed Phase Kieselgel (RP8); Methanol/5%ige wäßrige Kochsalzlösung = 6:4)

| | | | | |
|---|---|---|---|---|
| Ber.: | C 62,85 | H 6,70 | N 8,46 | Cl 7,49 |
| Gef.: | 62,88 | 6,76 | 8,45 | 7,52 |

Massenspektrum: $(M+H)^+$ = 450

Analog werden erhalten:

(1) (3S,5S)-5-[(4'-Amidino-4-biphenylyl)oxymethyl]-3-[(cyclohexylmethyloxycarbonyl)methyl]-2-pyrrolidinon x 1,08 HCl x 0,5 Wasser

$R_f$-Wert: 0,18 (Reversed Phase Kieselgel (RP8); Methanol/5%ige wäßrige Kochsalzlösung = 6:4)

| | | | | |
|---|---|---|---|---|
| Ber.: | C 63,34 | H 6,91 | N 8,21 | Cl 7,48 |
| Gef.: | 63,16 | 7,01 | 8,29 | 7,61 |

Massenspektrum: $(M+H)^+$ = 464

(2) (3S,5S)-5-[(4'-Amidino-4-biphenylyl)oxymethyl]-3-[(cyclopentyloxycarbonyl)methyl]-2-pyrrolidinon x 1,05 HCl x 0,5 Wasser

$R_f$-Wert: 0,27 (Reversed Phase Kieselgel (RP8); Methanol/5%ige wäßrige Kochsalzlösung = 6:4)

| | | | | |
|---|---|---|---|---|
| Ber.: | C 62,19 | H 6,48 | N 8,70 | Cl 7,71 |
| Gef.: | 62,05 | 6,70 | 8,82 | 7,79 |

Massenspektrum: $(M+H)^+ = 436$

(3) (3S,5S)-5-[(4'-Amidino-4-biphenylyl)oxymethyl]-3-[(cycloheptyloxycarbonyl)methyl]-2-pyrrolidinon x 1,1 HCl x 1 Wasser

$R_f$-Wert: 0,16 (Reversed Phase Kieselgel (RP8); Methanol/5%ige wäßrige Kochsalzlösung = 6:4)

| | | | | |
|---|---|---|---|---|
| Ber.: | C 62,16 | H 6,97 | N 8,05 | Cl 7,48 |
| Gef.: | 62,33 | 7,15 | 7,96 | 7,63 |

(4) (3S,5S)-5-[(4'-Amidino-4-biphenylyl)oxymethyl]-3-[(cyclooctyloxycarbonyl)methyl]-2-pyrrolidinon-hydrochlorid

(5) (3S,5S)-5-[(4'-Amidino-4-biphenylyl)oxymethyl]-3-[[(N-methylpiperidin-4-yl)oxycarbonyl]methyl]-2-pyrrolidinon-dihydrochlorid

(6) (3S,5S)-5-[(4'-Amidino-4-biphenylyl)oxymethyl]-3-[[[(tetrahydrofuran-2-yl)methyl]oxycarbonyl]methyl]-2-pyrrolidinon-hydrochlorid

(7) (3S,5S)-5-[(4'-Amidino-4-biphenylyl)oxymethyl]-3-[[[2-(cyclohexyl)ethyl]oxycarbonyl]methyl]-2-pyrrolidinon-hydrochlorid

(8) (3S,5S)-5-[(4'-Amidino-4-biphenylyl)oxymethyl]-3-[[(2-cyclopentylmethyl)oxycarbonyl]methyl]-2-pyrrolidinon-hydrochlorid

(9) (3S,5S)-5-[(4'-Amidino-4-biphenylyl)oxymethyl]-3-[[(cycloheptylmethyl)oxycarbonyl]methyl]-2-pyrrolidinon-hydrochlorid

(10) (3S,5S)-5-[(4'-Amidino-4-biphenylyl)oxymethyl]-3-[[(N-methylpiperidin-3-yl)oxycarbonyl]methyl]-2-pyrrolidinon-dihydrochlorid

(11) (3S,5S)-5-[(4'-Amidino-4-biphenylyl)oxymethyl]-3-[[(N-ethylpiperidin-4-yl)oxycarbonyl]methyl]-2-pyrrolidinon-dihydrochlorid

(12) (3S,5S)-5-[(4'-Amidino-4-biphenylyl)oxymethyl]-3-[[(N-methylpyrrolidin-2-yl)methyl]oxycarbonyl]methyl]-2-pyrrolidinon-dihydrochlorid

(13) (3S,5S)-5-[(4'-Amidino-4-biphenylyl)oxymethyl]-3-[[(tetrahydrofuran-3-yl)oxycarbonyl]methyl]-2-pyrrolidinon-hydrochlorid

(14) (3S,5S)-5-[(4'-Amidino-4-biphenylyl)oxymethyl]-3-[[(2-methylcyclohexyl)oxycarbonyl]methyl]-2-pyrrolidinon-hydrochlorid

(15) (3S,5S)-5-[(4'-Amidino-4-biphenylyl)oxymethyl]-3-[[(3-methylcyclohexyl)oxycarbonyl]methyl]-2-pyrrolidinon-hydrochlorid

(16) (3S,5S)-5-[(4'-Amidino-4-biphenylyl)oxymethyl]-3-[[(4-methylcyclohexyl)oxycarbonyl]methyl]-2-pyrrolidinon-hydrochlorid

(17) (3S,5S)-5-[(4'-Amidino-4-biphenylyl)oxymethyl]-3-[[(3,5-dimethylcyclohexyl)oxycarbonyl]methyl]-2-pyrrolidinon-hydrochlorid

(18) (3S,5S)-5-[(4'-Amidino-4-biphenylyl)oxymethyl]3-[[(3,3,5-trimethylcyclohexyl)oxycarbonyl]methyl]-2-pyrrolidinon-hydrochlorid

(19) (3S,5S)-5-[(4'-Amidino-4-biphenylyl)oxymethyl]-3-[[(3,3,5,5-tetramethylcyclohexyl)oxycarbonyl]methyl]-2-pyrrolidinon-hydrochlorid

(20) (3S,5S)-5-[(4'-Amidino-4-biphenylyl)oxymethyl]3-[[(4-ethylcyclohexyl)oxycarbonyl]methyl]-2-pyrrolidinon-hydrochlorid

(21) (3S,5S)-5-[(4'-Amidino-4-biphenylyl)oxymethyl]-3-[[(4-isopropylcyclohexyl)oxycarbonyl]methyl]-2-pyrrolidinon-hydrochlorid

(22) (3S,5S)-5-[(4'-Amidino-4-biphenylyl)oxymethyl]-3-[[(4-tert.butylcyclohexyl)oxycarbonyl]methyl]-2-pyrrolidinon-hydrochlorid

(23) (3S,5S)-5-[(4'-Amidino-4-biphenylyl)oxymethyl]-3-[(menthyloxycarbonyl)methyl]-2-pyrrolidinon-hydrochlorid

(24) (3S,5S)-5-[(4'-Amidino-4-biphenylyl)oxymethyl]-3-[[(4-methoxycyclohexyl)oxycarbonyl]methyl]-2-pyrrolidinon-hydrochlorid

(25) (3S,5S)-5-[(4'-Amidino-4-biphenylyl)oxymethyl]-3-[[(4-ethoxycyclohexyl)oxycarbonyl]methyl]-2-pyrrolidinon-hydrochlorid

(26) (3S,5S)-5-[(4'-Amidino-4-biphenylyl)oxymethyl]-3-[[(4-dimethylaminocyclohexyl)oxycarbonyl]methyl]-2-pyrrolidinon-dihydrochlorid

(27) (3S,5S)-5-[(4'-Amidino-4-biphenylyl)oxymethyl]-3-[[(4-trifluormethylcyclohexyl)oxycarbonyl]methyl]-2-pyrrolidinon-hydrochlorid

(28) (3S,5S)-5-[(4'-Amidino-4-biphenylyl)oxymethyl]-3-[[[(3-methylnorbornan-2-yl)methyl]oxycarbonyl]methyl]-2-pyrrolidinon-hydrochlorid

(29) (3S,5S)-5-[(4'-Amidino-4-biphenylyl)oxymethyl]-3-[[(2-norbornyl)oxycarbonyl]methyl]-2-pyrrolidinon-hydrochlorid

(30) (3S,5S)-5-[(4'-Amidino-4-biphenylyl)oxymethyl]-3-[(myrtanyloxycarbonyl)methyl]-2-pyrrolidinon-hydrochlorid

(31) (3S,5S)-5-[(4'-Amidino-4-biphenylyl)oxymethyl]-3-[[[2-(6,6-dimethyl-bicyclo[3.1.1]hept-2-yl)ethyl]oxycarbonyl]methyl]-2-pyrrolidinon-hydrochlorid

(32) (3S,5S)-5-[(4'-Amidino-4-biphenylyl)oxymethyl]-3-[(cinnamyloxycarbonyl)methyl]-2-pyrrolidinon-hydrochlorid

(33) (3S,5S)-5-[(4'-Amidino-4-biphenylyl)oxymethyl]-3-[[(2-indanyl)oxycarbonyl]methyl]-2-pyrrolidinon-hydrochlorid

## Beispiel 5

(3S,5S)-3-[(Cyclohexyloxycarbonyl)methyl]-5-[(4'-methoxycarbonylamidino-4-biphenylyl)oxymethyl]-2-pyrrolidinon

Zu einem Gemisch aus 1,1 g (3S,5S)-5-[(4'-Amidino-4-biphenylyl)oxymethyl]-3-[(cyclohexyloxycarbonyl)methyl]-2-pyrrolidinon x 1,05 HCl x 0,5 Wasser in 30 ml Tetrahydrofuran und 5 ml Wasser werden 0,2 ml Chlorameisensäuremethylester gegeben und dann unter Rühren 9,6 ml 0,5N Natronlauge zugetropft. Das Reaktionsgemisch wird eingeengt, mit Eiswasser versetzt und der Feststoff abgesaugt und getrocknet. Nach Chromatographie über eine Kieselgelsäule mit Essigester wird ein Feststoff erhalten, der mit 20 ml Essigester aufgekocht wird. Nach dem Abkühlen wird abgesaugt, mit Diethylether gewaschen und getrocknet.

Ausbeute: 570 mg (50 % der Theorie),
$R_f$-Wert: 0,27 (Kieselgel; Essigester)

| | | | |
|---|---|---|---|
| Ber.: | C 66,26 | H 6,55 | N 8,28 |

(fortgesetzt)

| | | | |
|---|---|---|---|
| Gef.: | 65,96 | 6,54 | 8,37 |

Massenspektrum: $(M+H)^+$ = 508

Analog werden erhalten:

(1) (3S,5S)-3-[(Cyclohexylmethyloxycarbonyl)methyl]-5-[(4'-ethoxycarbonylamidino-4-biphenylyl)oxymethyl]-2-pyrrolidinon

(2) (3S,5S)-3-[(Cyclopentyloxycarbonyl)methyl]-5-[(4'-methoxycarbonylamidino-4-biphenylyl)oxymethyl]-2-pyrrolidinon

(3) (3S,5S)-3-[(Cycloheptyloxycarbonyl)methyl]-5-[(4'-methoxycarbonylamidino-4-biphenylyl)oxymethyl]-2-pyrrolidinon

(4) (3S,5S)-3-[(Cyclooctyloxycarbonyl)methyl]-5-[(4'-methoxycarbonylamidino-4-biphenylyl)oxymethyl]-2-pyrrolidinon

Beispiel 6

(3S,5S)-5-[[4'-(O,O'-Diethylphosphono)amidino-4-biphenylyl]oxymethyl]-3-[(methoxycarbonyl)methyl]-2-pyrrolidinon

Zu einem Gemisch aus 1,1 g (3S,5S)-5-[(4'-Amidino-4-biphenylyl)oxymethyl]-3-[(methoxycarbonyl)methyl]-2-pyrrolidinon-toluolsulfonat in 20 ml Tetrahydrofuran und 2 ml Wasser werden 380 mg Phosphorsäure-diethylester-chlorid zugegeben und dann unter Rühren 8,4 ml 0,5N Natronlauge zugetropft. Es wird nochmals etwas Phosphorsäure-diethylester-chlorid und Natronlauge zugegeben und weitergerührt. Das organische Lösungsmittel wird abrotiert, die wäßrige Phase abdekantiert und der ölige Rückstand durch Chromatographie über eine Kieselgelsäule mit Methylenchlorid/Methanol (95:5) gereinigt.

Ausbeute: 0,47 g (45 % der Theorie),
$R_f$-Wert: 0,13 (Reversed Phase Kieselgel (RP8); Methanol/5%ige wäßrige Kochsalzlösung = 6:4)

| | | | |
|---|---|---|---|
| Ber.: | C 58,02 | H 6,23 | N 8,12 |
| Gef.: | 57,88 | 6,42 | 8,30 |

Massenspektrum: $M^+$ = 517

Beispiel 7

Trockenampulle mit 2,5 mg Wirkstoff pro 1 ml

Zusammensetzung:

| | |
|---|---|
| Wirkstoff | 2,5 mg |
| Mannitol | 50,0 mg |
| Wasser für Injektionszwecke ad | 1,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 8

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 9

Tablette mit 350 mg Wirkstoff

Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 10

Kapseln mit 50 mg Wirkstoff

Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 11

Kapseln mit 350 mg Wirkstoff

Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

**1.** Cyclische Iminoderivate der allgemeinen Formel

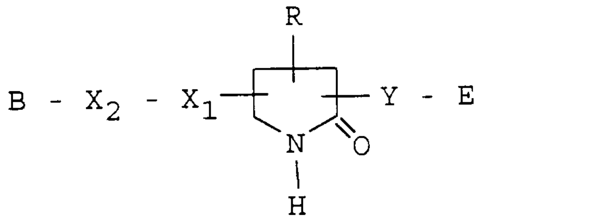

, (I)

in der mit der Maßgabe, daß

(i) B eine $R_1$-CO-O-($R_2CR_3$)-O-CO-NH-C(=NH)- oder ($R_4$O)PO(O$R_5$)-NH-C(=NH)-Gruppe oder E eine $R_6$-CO-O-CH$R_7$-O-CO- oder $R_8$O-CO-Gruppe oder

(ii) B eine $R_1$-CO-O-($R_2CR_3$)-O-CO-NH-C(=NH)- oder ($R_4$O)PO(O$R_5$)-NH-C(=NH)-Gruppe und E eine $R_6$-CO-O-CH$R_7$-O-CO- oder $R_8$O-CO-Gruppe darstellen, in denen

$R_1$ eine Alkylgruppe mit 1 bis 4 Kohlenstoffatomen,

$R_2$ ein Wasserstoffatom oder eine Methylgruppe,

$R_3$ ein Wasserstoffatom,

$R_4$ und $R_5$, die gleich oder verschieden sein können, Methyl- oder Ethylgruppen,

$R_6$ eine Alkylgruppe mit 1 bis 4 Kohlenstoffatomen, eine Cyclohexyl- oder Phenylgruppe, eine Alkoxygruppe mit 1 bis 4 Kohlenstoffatomen oder eine Cycloalkoxygruppe mit 5 bis 7 Kohlenstoffatomen,

$R_7$ ein Wasserstoffatom oder eine Methylgruppe und

$R_8$ eine Cycloalkyl-, Cycloalkylmethyl- oder Cycloalkylethylgruppe mit jeweils 5 bis 8 Kohlenstoffatomen im Cycloalkylteil, wobei die vorstehend erwähnten Cycloalkylteile zusätzlich durch eine Alkylgruppe mit 1 bis 4 Kohlenstoffatomen oder durch eine Alkylgruppe mit 1 bis 4 Kohlenstoffatomen und 1 bis 3 Methylgruppen, durch eine Methoxy-, Ethoxy-, Dimethylamino- oder Diethylaminogruppe substituiert sein können und außerdem in den vorstehend erwähnten Cycloalkylteilen jeweils eine Methylengruppe durch ein Sauerstoffatom, durch eine Methylimino- oder Ethyliminogruppe mit der Maßgabe ersetzt sein kann, daß sich zwischen dem Ringheteroatom und dem nächsten Heteroatom mindestens 2 Kohlenstoffatome befinden,

eine Cyclohexenyl- oder Cyclohexenylmethylgruppe, wobei die vorstehend erwähnten Cyclohexenylteile zusätzlich durch eine Methylgruppe substituiert sein können, mit der Maßgabe, daß die vorstehend erwähnten Cyclohexenylteile nicht über ein Kohlenstoffatom, von dem eine Doppelbindung ausgeht, mit dem Sauerstoffatom der benachbarten -O-CO-Gruppe verknüpft sind,

eine Bicycloalkyl- oder Bicycloalkylalkylgruppe mit jeweils 6 bis 8 Kohlenstoffatomen im Bicycloalkylteil und 1 oder 2 Kohlenstoffatomen im Alkylteil, wobei die vorstehend erwähnten Bicycloalkylteile zusätzlich durch 1 bis 3 Methylgruppen substituiert sein können,

eine Bicycloalkenyl- oder Bicycloalkenylalkylgruppe mit jeweils 7 oder 8 Kohlenstoffatomen im Bicycloalkenylteil und 1 oder 2 Kohlenstoffatomen im Alkylteil, wobei die vorstehend erwähnten Bicycloalkenylteile zusätzlich durch 1 bis 3 Methylgruppen subtituiert sein können, mit der Maßgabe, daß die vorstehend erwähnten Bicycloalkenylteile nicht über ein Kohlenstoffatom, von dem eine Doppelbindung ausgeht, mit dem Sauerstoffatom der benachbarten -O-CO-Gruppe verknüpft sind, eine Benzocycloalkylgruppe mit insgesamt 9 oder 10 Kohlenstoffatomen,

eine Alkenyl- oder Alkinylgruppe mit jeweils 3 bis 5 Kohlenstoffatomen mit der Maßgabe, daß die vorstehend erwähnten Alkenyl- oder Alkinylgruppen nicht über ein Kohlenstoffatom, von dem eine Doppel- oder Dreifachbindung ausgeht, mit dem Sauerstoffatom der benachbarten -O-CO-Gruppe verknüpft sind, oder

eine Cinnamylgruppe bedeuten,

R ein an ein Kohlenstoffatom des 2-Pyrrolidinonringes gebundenes Wasserstoffatom oder eine Methylgruppe,

B eine $HNR_{11}$-C(=NH)-Gruppe, in der $R_{11}$ ein Wasserstoffatom, eine Methoxycarbonyl-, Ethoxycarbonyl- oder Benzyloxycarbonylgruppe darstellt, eine $R_1$-CO-O-($R_2CR_3$)-O-CO-NH-C(=NH)- oder ($R_4$O)PO($OR_5$)-NH-C(=NH)-Gruppe, in denen $R_1$ bis $R_5$ wie vorstehend erwähnt definiert sind,

Y eine Methylengruppe,

E eine Carboxygruppe, eine Alkoxycarbonylgruppe mit insgesamt 2 bis 6 Kohlenstoffatomen, eine $R_6$-CO-O-$CHR_7$-O-CO- oder $R_8$O-CO-Gruppe, wobei $R_6$ bis $R_8$ wie vorstehend erwähnt definiert sind,

$X_1$ eine Methylengruppe, die, sofern diese nicht an das Ringstickstoffatom des 2-Pyrrolidinonringes gebunden ist, über ein Sauerstoffatom, eine Sulfonyl-, -CO-NH- oder -$SO_2$-NH-Gruppe an die benachbarte $X_2$-Gruppe gebunden ist, wobei die Carbonyl- und Sulfonylgruppe der vorstehend erwähnten -CO-NH- und -$SO_2$-NH-Gruppen mit dem Rest $X_2$ verknüpft ist, und

$X_2$ eine Biphenylylengruppe, die durch ein Fluor- oder Chloratom, durch eine Methyl- oder Trifluormethylgruppe oder durch eine weitere Methylgruppe substituiert sein kann, bedeuten,
deren Stereoisomere, deren Tautomere, deren Gemische und deren Salze.

**2.** Cyclische Iminoderivate der allgemeinen Formel I gemäß Anspruch 1, in denen mit der Maßgabe, daß

(i) B eine $R_1$-CO-O-($R_2CR_3$)-O-CO-NH-C(=NH)- oder ($R_4$O)PO($OR_5$)-NH-C(=NH)-Gruppe oder
E eine $R_6$-CO-O-$CHR_7$-O-CO- oder $R_8$O-CO-Gruppe oder

(ii) B eine $R_1$-CO-O-($R_2CR_3$)-O-CO-NH-C(=NH)- oder ($R_4$O)PO($OR_5$)-NH-C(=NH)-Gruppe und
E eine $R_6$-CO-O-$CHR_7$-O-CO- oder $R_8$O-CO-Gruppe darstellen, in denen

$R_1$ eine Methylgruppe,

$R_2$ ein Wasserstoffatom oder eine Methylgruppe,

$R_3$ ein Wasserstoffatom,

$R_4$ und $R_5$ jeweils eine Ethylgruppe,

$R_6$ eine tert.Butylgruppe, eine Ethoxy- oder Cyclohexyloxygruppe,

$R_7$ ein Wasserstoffatom oder eine Methylgruppe und

$R_8$ eine Cyclopentyl-, Cyclohexyl-, Cycloheptyl- oder Cyclohexylmethylgruppe bedeuten,

R ein Wasserstoffatom,

B eine $HNR_{11}$-C(=NH)-Gruppe, in der $R_{11}$ ein Wasserstoffatom, eine Methoxycarbonyl- oder Benzyloxycarbonylgruppe darstellt, eine $R_1$-CO-O-($R_2CR_3$)-O-CO-NH-C(=NH)- oder ($R_4$O)PO($OR_5$)-NH-C(=NH)-Gruppe, in denen $R_1$ bis $R_5$ wie vorstehend erwähnt definiert sind,

Y eine Methylengruppe,

E eine Methoxycarbonylgruppe, eine $R_6$-CO-O-$CHR_7$-O-CO- oder $R_8$O-CO-Gruppe, wobei $R_6$ bis $R_8$ wie vorstehend erwähnt definiert sind,

$X_1$ und $X_2$ zusammen eine 4-Biphenylylenoxymethylengruppe, wobei die Gruppe B in 4'-Stellung mit der 4-Biphenylylenoxymethylengruppe verknüpft ist, und die Methylengruppe der 4-Biphenylylenoxymethylengruppe mit einem Kohlenstoffatom des 2-Pyrrolidinonringes verknüpft ist, bedeuten,
deren Stereoisomere, deren Tautomere, deren Gemische und deren Salze.

**3.** Cyclische Iminoderivate der allgemeinen Formel I gemäß Anspruch 1, in denen A, B, E, $X_1$, $X_2$ und Y wie im Anspruch 2 definiert sind,

der Rest -Y-E in Position 3 und
der Rest B-$X_2$-$X_1$- in Position 5 des 2-Pyrrolidinonringes steht,
deren Stereoisomere, deren Tautomere, deren Gemische und deren Salze.

**4.** Folgende Verbindungen der allgemeinen Formel I gemäß Anspruch 1:

(3S,5S)-5-[(4'-Acetoxymethyloxycarbonylamidino-4-biphenylyl)oxymethyl]-3-[(methoxycarbonyl)methyl]-2-pyrrolidinon,

(3S,5S)-5-[[4'-(1-Acetoxyethyl)oxycarbonylamidino-4-biphenylyl]oxymethyl]-3-[(methoxycarbonyl)methyl]-2-pyrrolidinon,

(3S,5S)-5-[(4'-Benzyloxycarbonylamidino-4-biphenylyl)oxymethyl]-3-[[[1-(cyclohexyloxycarbonyloxy)ethyl]oxycarbonyl]-methyl]-2-pyrrolidinon,

(3S,5S)-5-[(4'-Benzyloxycarbonylamidino-4-biphenylyl)-oxymethyl]-3-[[[1-(ethyloxycarbonyloxy)ethyl]oxycarbonyl]-methyl]-2-pyrrolidinon,

(3S,5S)-5-[(4'-Benzyloxycarbonylamidino-4-biphenylyl)-oxymethyl]-3-[(pivaloyloxymethyloxycarbonyl)methyl]-2-pyrrolidinon,

(3S,5S)-5-[(4'-Methoxycarbonylamidino-4-biphenylyl)oxymethyl]-3-[(pivaloyloxymethyloxycarbonyl)methyl]-2-pyrrolidinon,

(3S,5S)-5-[(4'-Amidino-4-biphenylyl)oxymethyl]-3-[[[1-(cyclohexyloxycarbonyloxy)ethyl]oxycarbonyl]methyl]-2-pyrrolidinon,

(3S,5S)-5-[(4'-Amidino-4-biphenylyl)oxymethyl]-3-[[[1-(ethyloxycarbonyloxy)ethyl]oxycarbonyl]methyl]-2-pyrrolidinon,

(3S,5S)-5-[(4'-Amidino-4-biphenylyl)oxymethyl]-3-[(pivaloyloxymethyloxycarbonyl)methyl]-2-pyrrolidinon,

(3S,5S)-5-[(4'-Amidino-4-biphenylyl)oxymethyl]-3-[(cyclohexyloxycarbonyl)methyl]-2-pyrrolidinon,

(3S,5S)-5-[(4'-Amidino-4-biphenylyl)oxymethyl]-3-[(cyclohexylmethyloxycarbonyl)methyl]-2-pyrrolidinon,

(3S,5S)-5-[(4'-Amidino-4-biphenylyl)oxymethyl]-3-[(cyclopentyloxycarbonyl)methyl]-2-pyrrolidinon,

(3S,5S)-5-[(4'-Amidino-4-biphenylyl)oxymethyl]-3-[(cycloheptyloxycarbonyl)methyl]-2-pyrrolidinon,

(3S,5S)-3-[(Cyclohexyloxycarbonyl)methyl]-5-[(4'-methoxy-carbonylamidino-4-biphenylyl)oxymethyl]-2-pyrrolidinon und

(3S,5S)-5-[[4'-(O,O'-Diethylphosphono)amidino-4-biphenylyl]oxymethyl]-3-[(methoxycarbonyl)methyl]-2-pyrrolidinon
sowie deren Salze.

**5.** Physiologisch verträgliche Salze der Verbindungen nach mindestens einem der Ansprüche 1 bis 4 mit anorganischen oder organischen Säuren oder Basen.

6. Arzneimittel, enthaltend eine Verbindung nach mindestens einem der Ansprüche 1 bis 4 oder ein physiologisch verträgliches Salz gemäß Anspruch 5 neben gegebenenfalls einem oder mehreren inerten Trägerstoffen und/oder Verdünnungsmitteln.

7. Verwendung einer Verbindung nach mindestens einem der Ansprüche 1 bis 5 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

8. Verfahren zur Herstellung eines Arzneimittels gemäß Anspruch 6, dadurch gekennzeichnet, daß auf nichtchemischem Wege eine Verbindung nach mindestens einem der Ansprüche 1 bis 5 in einen oder mehrere inerte Trägerstoffe und/oder Verdünnungsmittel eingearbeitet wird.

9. Verfahren zur Herstellung der cyclischen Iminoderivate gemäß den Ansprüchen 1 bis 5, dadurch gekennzeichnet, daß

a) zur Herstellung von Verbindungen der allgemeinen Formel I, in der B eine $R_1$-CO-O-($R_2CR_3$)-O-CO-NH-C(=NH)- oder $HNR_{11}$-C(=NH)-Gruppe darstellt, wobei $R_1$ bis $R_3$ wie in den Ansprüchen 1 bis 4 definiert sind, und $R_{11}$ eine Methoxycarbonyl-, Ethoxycarbonyl- oder Benzyloxycarbonylgruppe bedeuten, eine Verbindung der allgemeinen Formel

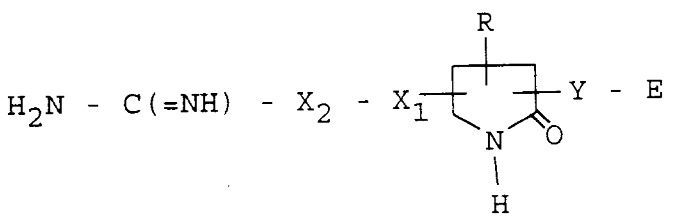

, (II)

in der

R, E, $X_1$, $X_2$ und Y wie in den Ansprüchen 1 bis 4 definiert sind, mit einer Verbindung der allgemeinen Formel

$$Z_1 - B_1 , \quad \text{(III)}$$

in der
$B_1$ eine Methoxycarbonyl-, Ethoxycarbonyl- oder Benzyloxycarbonylgruppe oder eine $R_1$-CO-O-($R_2CR_3$)-O-CO-Gruppe, in der $R_1$ bis $R_3$ wie in den Ansprüchen 1 bis 4 definiert sind, und
$Z_1$ eine nukleophile Austrittsgruppe bedeuten, umgesetzt wird oder

b) zur Herstellung von Verbindungen der allgemeinen Formel I, in der E eine Alkoxycarbonylgruppe mit insgesamt 2 bis 6 Kohlenstoffatomen, eine $R_6$-CO-O-$CHR_7$-O-CO- oder $R_8$O-CO-Gruppe darstellt, wobei $R_6$ bis $R_8$ wie in den Ansprüchen 1 bis 4 definiert sind, eine Verbindung der allgemeinen Formel

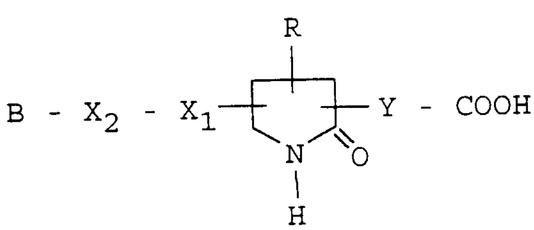

, (IV)

in der

R, B, $X_1$, $X_2$ und Y wie in den Ansprüchen 1 bis 4 definiert sind, mit einer Verbindung der allgemeinen For-

mel

$$Z_2 - E_1 , \qquad (V)$$

in der
$E_1$ eine Alkylgruppe mit insgesamt 1 bis 5 Kohlenstoffatomen, eine $R_6$-CO-O-$CHR_7$- oder $R_8$-Gruppe darstellt, wobei $R_6$ bis $R_8$ wie in den Ansprüchen 1 bis 4 definiert sind, und
$Z_2$ eine nukleophile Austrittsgruppe darstellen, umgesetzt wird oder

c) zur Herstellung von Verbindungen der allgemeinen Formel I, in der E eine Alkoxycarbonylgruppe mit insgesamt 2 bis 6 Kohlenstoffatomen oder eine $R_8$O-CO-Gruppe darstellt, wobei $R_8$ wie in den Ansprüchen 1 bis 4 definiert ist, eine Verbindung der allgemeinen Formel

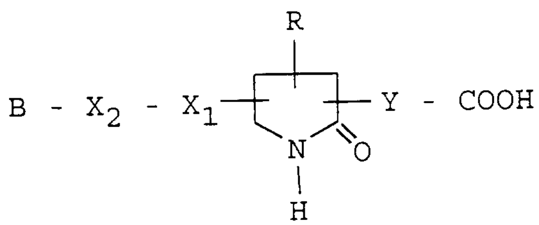
, (IV)

in der

R, B, $X_1$, $X_2$ und Y wie in den Ansprüchen 1 bis 4 definiert sind, mit einer Verbindung der allgemeinen Formel

$$HO - E_2 , \qquad (VI)$$

in der
$E_2$ eine Alkylgruppe mit 1 bis 5 Kohlenstoffatomen oder eine $R_8$-Gruppe, wobei $R_8$ wie in den Ansprüchen 1 bis 4 definiert ist, darstellt, verestert wird oder

d) zur Herstellung von Verbindungen der allgemeinen Formel I, in der B eine $(R_4O)PO(OR_5)$-NH-C(=NH)-Gruppe darstellt, wobei $R_4$ und $R_5$ wie in den Ansprüchen 1 bis 4 definiert sind, eine Verbindung der allgemeinen Formel

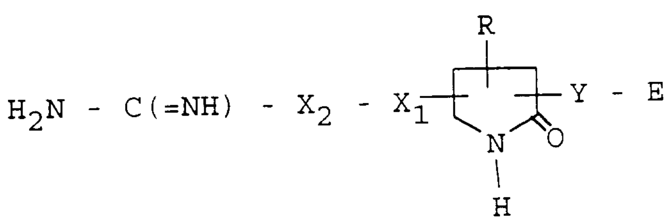
, (II)

in der

R, E, $X_1$, $X_2$ und Y wie in den Ansprüchen 1 bis 4 definiert sind, mit einer Verbindung der allgemeinen Formel

$$(R_4O)PO(OR_5)-Z_3 , \qquad (VII)$$

in der
$R_4$ und $R_5$ wie in den Ansprüchen 1 bis 4 definiert sind und
$Z_3$ eine nukleophile Austrittsgruppe bedeuten, umgesetzt wird und

erforderlichenfalls ein während den Umsetzungen zum Schutze von reaktiven Gruppen verwendeter Schutzrest abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

## Claims

1. Cyclic imino derivatives of general formula

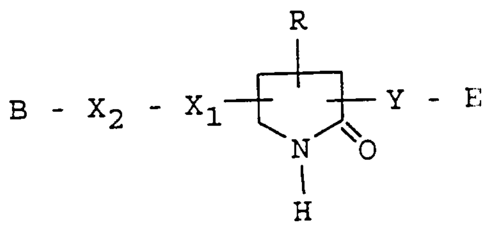

, (I)

wherein with the proviso that

(i) B denotes an $R_1$-CO-O-($R_2CR_3$)-O-CO-NH-C(=NH)- or ($R_4$O)PO($OR_5$)-NH-C(=NH)- group or E denotes an $R_6$-CO-O-$CHR_7$-O-CO- or $R_8$O-CO- group or

(ii) B denotes an $R_1$-CO-O-($R_2CR_3$)-O-CO-NH-C(=NH)- or ($R_4$O)PO($OR_5$)-NH-C(=NH)- group and E denotes an $R_6$-CO-O-$CHR_7$-O-CO- or $R_8$O-CO- group, wherein

$R_1$ denotes a $C_{1-4}$-alkyl group,

$R_2$ denotes a hydrogen atom or a methyl group,

$R_3$ denotes a hydrogen atom,

$R_4$ and $R_5$, which may be identical or different, represent methyl or ethyl groups,

$R_6$ denotes a $C_{1-4}$-alkyl group, a cyclohexyl or phenyl group, a $C_{1-4}$-alkoxy group or a $C_{5-7}$-cycloalkoxy group,

$R_7$ denotes a hydrogen atom or a methyl group and

$R_8$ denotes a cycloalkyl, cycloalkylmethyl or cycloalkylethyl group each having 5 to 8 carbon atoms in the cycloalkyl moiety, in which the above-mentioned cycloalkyl moieties may additionally be substituted by a $C_{1-4}$-alkyl group or by a $C_{1-4}$-alkyl group and 1 to 3 methyl groups, by a methoxy, ethoxy, dimethylamino, diethylamino or trifluoromethyl group and moreover, in the above-mentioned cycloalkyl moieties, a methylene group may be replaced by an oxygen atom or by a methylimino or ethylimino group, with the proviso that there are at least two carbon atoms between the ring heteroatom and the next heteroatom,

a cyclohexenyl or cyclohexenylmethyl group, in which the above-mentioned cyclohexenyl moieties may additionally be substituted by a methyl group, with the proviso that the above-mentioned cyclohexenyl moieties are not linked to the oxygen atom of the adjacent -O-CO- group via a carbon atom from which a double bond starts,

a bicycloalkyl or bicycloalkylalkyl group each having 6 to 8 carbon atoms in the bicycloalkyl moiety and 1 or 2 carbon atoms in the alkyl moiety, whilst the above-mentioned bicycloalkyl moieties may additionally be substituted by 1 to 3 methyl groups,

a bicycloalkenyl or bicycloalkenylalkyl group each having 7 or 8 carbon atoms in the bicycloalkenyl moiety and 1 or 2 carbon atoms in the alkyl moiety, whilst the above-mentioned bicycloalkenyl moieties may additionally be substituted by 1 to 3 methyl groups, with the proviso that the above-mentioned bicycloalkenyl moieties are not linked to the oxygen atom of the adjacent -O-CO- group via a carbon atom from which a double bond starts,

a benzocycloalkenyl group having a total of 9 or 10 carbon atoms,

an alkenyl or alkynyl group each having 3 to 5 carbon atoms, with the proviso that the above-mentioned alkenyl or alkynyl groups are not linked to the oxygen atom of the adjacent -O-CO- group via a carbon atom from which a double or triple bond starts, or

a cinnamyl group,

R denotes a hydrogen atom or a methyl group bound to a carbon atom of the 2-pyrrolidinone ring,

B denotes an $HNR_{11}$-C(=NH)- group wherein $R_{11}$ represents a hydrogen atom, a methoxycarbonyl, ethoxycarbonyl, benzyloxycarbonyl, $R_1$-CO-O-($R_2CR_3$)-O-CO-NH-C(=NH)- or ($R_4$O)PO($OR_5$)-NH-C(=NH)- group, wherein $R_1$ to $R_5$ are defined as hereinbefore,

Y denotes a methylene group,

E denotes a carboxy group, an alkoxycarbonyl group having a total of 2 to 6 carbon atoms, an $R_6$-CO-O-$CHR_7$-O-CO- or $R_8$O-CO- group, wherein $R_6$ to $R_8$ are defined as hereinbefore,

$X_1$ denotes a methylene group which, provided it is not bound to the ring nitrogen atom of the pyrrolidinone ring, is bound to the adjacent $X_2$ group via an oxygen atom or a sulphonyl, -CO-NH- or -$SO_2$-NH- group, in which the carbonyl and sulphonyl groups of the above-mentioned -CO-NH- and -$SO_2$-NH- groups are linked to the group $X_2$, and

$X_2$ denotes a biphenylylene group which may be substituted by a fluorine or chlorine atom, by a methyl or trifluoromethyl group or by another methyl group,
the stereoisomers thereof, the tautomers thereof, the mixtures and salts thereof.

**2.** Cyclic imino derivatives of general formula I according to claim 1 wherein with the proviso that

(i) B denotes an $R_1$-CO-O-($R_2CR_3$)-O-CO-NH-C(=NH)- or ($R_4$O)PO($OR_5$)-NH-C(=NH)- group or
E denotes an $R_6$-CO-O-$CHR_7$-O-CO- or $R_8$O-CO- group or

(ii) B denotes an $R_1$-CO-O-($R_2CR_3$)-O-CO-NH-C(=NH)- or ($R_4$O)PO($OR_5$)-NH-C(=NH)- group and
E denotes an $R_6$-CO-O-$CHR_7$-O-CO- or $R_8$O-CO- group, wherein

$R_1$ denotes a methyl group,

$R_2$ denotes a hydrogen atom or a methyl group,

$R_3$ denotes a hydrogen atom,

$R_4$ and $R_5$ each denote an ethyl group,

$R_6$ denotes a tert.butyl group or an ethoxy or cyclohexyloxy group,

$R_7$ denotes a hydrogen atom or a methyl group and

$R_8$ denotes a cyclopentyl, cyclohexyl, cycloheptyl or cyclohexylmethyl group,

R denotes a hydrogen atom,

B denotes an $HNR_{11}$-C(=NH)- group wherein $R_{11}$ represents a hydrogen atom, a methoxycarbonyl or benzyloxycarbonyl, $R_1$-CO-O-($R_2CR_3$)-O-CO-NH-C(=NH)- or ($R_4$O)PO($OR_5$)-NH-C(=NH)-group wherein $R_1$ to $R_5$ are defined as hereinbefore,

Y denotes a methylene group,

E denotes a methoxycarbonyl group, an $R_6$-CO-O-$CHR_7$-O-CO- or $R_8$O-CO- group, wherein $R_6$ to $R_8$ are as hereinbefore defined,

$X_1$ and $X_2$ together denote a 4-biphenylyleneoxymethylene group, wherein the group B in the 4'-position is linked to the 4-biphenylyleneoxymethylene group, and the methylene group of the 4-biphenylyleneoxymethylene group is linked to a carbon atom of the 2-pyrrolidinone ring,
the stereoisomers, the tautomers and the mixtures thereof and the salts thereof.

**3.** Cyclic imino derivatives of general formula I according to claim 1, wherein A, B, E, $X_1$, $X_2$ and Y are defined as in claim 2,

the group -Y-E is in position 3 and
the group B-$X_2$-$X_1$- is in position 5 of the 2-pyrrolidinone ring,
the stereoisomers and tautomers thereof, the mixtures thereof and salts thereof.

**4.** The following compounds of general formula I according to claim 1:

(3S,5S)-5-[(4'-acetoxymethyloxycarbonylamidino-4-biphenylyl)oxymethyl]-3-[(methoxycarbonyl)methyl]-2-pyrrolidinone,

(3S,5S)-5-[[4'-(1-acetoxyethyl)oxycarbonylamidino-4-biphenylyl]oxymethyl]-3-[(methoxycarbonyl)methyl]-2-pyrrolidinone,

(3S,5S)-5-[(4'-benzyloxycarbonylamidino-4-biphenylyl)oxymethyl]-3-[[[1-(cyclohexyloxycarbonyloxy)ethyl] oxycarbonyl]methyl]-2-pyrrolidinone,

(3S,5S)-5-[(4'-benzoyloxycarbonylamidino-4-biphenylyl)oxymethyl]-3-[[[1-(ethyloxycarbonyloxy)ethyl]oxycarbonyl]-methyl-2-pyrrolidinone,

(3S,5S)-5-[(4'-benzyloxycarbonylamidino-4-biphenylyl)oxymethyl]-3-[(pivaloyloxymethyloxycarbonyl)methyl]-2-pyrrolidinone,

(3S,5S)-5-[(4'-methoxycarbonylamidino-4-biphenylyl)oxymethyl]-3-[(pivaloyloxymethyloxycarbonyl)methyl]-2-pyrrolidinone,

(3S,5S)-5-[(4'-amidino-4-biphenylyl)oxymethyl]-3-[[[1-(cyclohexyloxycarbonyloxy)ethyl]oxycarbonyl]methyl]-2-pyrrolidinone,

(3S,5S)-5-[(4'-amidino-4-biphenylyl)oxymethyl]-3-[[[1-(ethyloxycarbonyloxy)ethyl]oxycarbonyl]methyl]-2-pyrrolidinone,

(3S,5S)-5-[(4'-amidino-4-biphenylyl)oxymethyl]-3-[(pivaloyloxymethyloxycarbonyl)methyl]-2-pyrrolidinone,

(3S,5S)-5-[(4'-amidino-4-biphenylyl)oxymethyl]-3-[(cyclohexyloxymethyloxycarbonyl)methyl]-2-pyrrolidinone,

(3S,5S)-5-[(4'-amidino-4-biphenylyl)oxymethyl]-3-[(cyclohexylmethyloxycarbonyl)methyl]-2-pyrrolidinone,

(3S,5S)-5-[(4'-amidino-4-biphenylyl)oxymethyl]-3-[(cyclopentyloxycarbonyl)methyl]-2-pyrrolidinone,

(3S,5S)-5-[(4'-amidino-4-biphenylyl)oxymethyl]-3-[(cycloheptyloxycarbonyl)methyl]-2-pyrrolidinone,

(3S,5S)-3-[(cyclohexyloxycarbonyl)methyl]-5-[(4'-methoxycarbonylamidino-4-biphenylyl)oxymethyl]-2-pyrrolidinone,

(3S,5S)-5-[[4'-(O,O'-diethylphosphono)amidino-4-biphenylyl]oxymethyl]-3-[(methoxycarbonyl)methyl]-2-pyrrolidinone,
and the salts thereof.

**5.** Physiologically acceptable salts of the compounds according to at least one of claims 1 to 4 with inorganic or organic acids or bases.

**6.** Pharmaceutical compositions containing a compound according to at least one of claims 1 to 4 or a physiologically acceptable salt according to claim 5, optionally together with one or more inert carriers and/or diluents.

**7.** Use of a compound according to at least one of claims 1 to 5 for preparing a pharmaceutical composition which is suitable for combating or preventing diseases in which smaller or larger cell aggregates occur or cell-matrix interactions are involved.

**8.** Process for preparing a pharmaceutical composition according to claim 6, characterised in that a compound according to at least one of claims 1 to 5 is incorporated in one or more inert carriers and/or diluents by a non-chemical method.

**9.** Process for preparing the cyclic imino derivatives according to claims 1 to 5, characterised in that

a) in order to prepare compounds of general formula I wherein B denotes an $R_1$-CO-O-($R_2CR_3$)-O-CO-NH-C(=NH)- or $HNR_{11}$-C(=NH)- group, wherein $R_1$ to $R_3$ are defined as in claims 1 to 4 and $R_{11}$ denotes a methoxycarbonyl, ethoxycarbonyl or benzyloxycarbonyl group, a compound of general formula

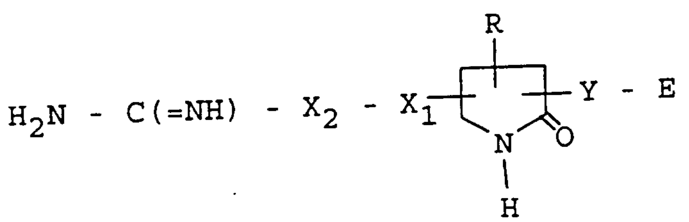
, (II)

wherein

R, E, $X_1$, $X_2$ and Y are defined as in claims 1 to 4, is reacted with a compound of general formula

$$Z_1 - B_1 \qquad (III)$$

wherein
$B_1$ denotes a methoxycarbonyl, ethoxycarbonyl or benzyloxycarbonyl group or an $R_1$-CO-O-($R_2CR_3$)-O-CO- group in which $R_1$ to $R_3$ are defined as in claims 1 to 4, and
$Z_1$ denotes a nucleophilic leaving group, or

b) in order to prepare compounds of general formula I wherein E denotes an alkoxycarbonyl group having a total of 2 to 6 carbon atoms, an $R_6$-CO-O-$CHR_7$-O-CO- or $R_8$O-CO-group, a compound of general formula

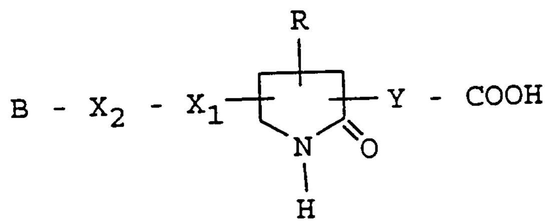

, (IV)

wherein

R, B, $X_1$, $X_2$ and Y are defined as in claims 1 to 4 is reacted with a compound of general formula

$$Z_2 - E_1 \qquad (V)$$

wherein
$E_1$ denotes a $C_{1-5}$-alkyl group, an $R_6$-CO-O-$CHR_7$- or $R_8$ group, wherein $R_6$ to $R_8$ are defined as in claims 1 to 4, and
$Z_2$ denotes a nucleophilic leaving group, or

c) in order to prepare compounds of general formula I wherein E denotes an alkoxycarbonyl group having a total of 2 to 6 carbon atoms, or an $R_8$O-CO- group, a compound of general formula

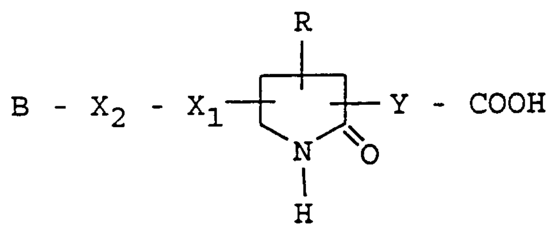

, (IV)

wherein

R, B, $X_1$, $X_2$ and Y are defined as in claims 1 to 4, is esterified with a compound of general formula

$$HO - E_2 \qquad (VI)$$

wherein
$E_2$ denotes a $C_{1-5}$-alkyl group or an $R_8$ group in which $R_8$ is defined as in claims 1 to 4, or

d) in order to prepare compounds of general formula I wherein B denotes an $(R_4O)PO(OR_5)$-NH-C(=NH)- group, in which $R_4$ and $R_5$ are defined as in claims 1 to 4, a compound of general formula

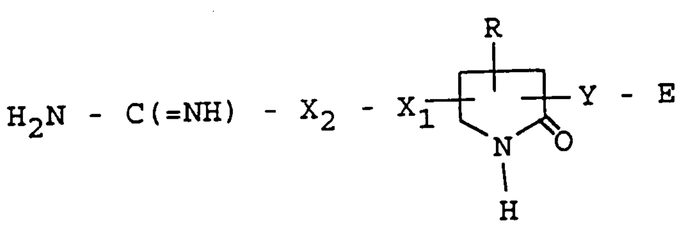

, (II)

wherein

R, E, $X_1$, $X_2$ and Y are defined as in claims 1 to 4, is reacted with a compound of general formula

$(R_4O)OP(OR_5)-Z_3$ (VII)

(wherein
$R_4$ and $R_5$ are defined as in claims 1 to 4 and
$Z_3$ denotes a nucleophilic leaving group, and

if necessary a protecting group used during the reactions to protect any reactive groups is cleaved and/or if desired a compound of general formula I thus obtained is resolved into the stereoisomers thereof and/or a compound of general formula I thus obtained is converted into the salts thereof, more particularly for pharmaceutical use into its physiologically acceptable salts with an inorganic or organic acid or base.

## Revendications

1. Dérivés imino cycliques de formule générale

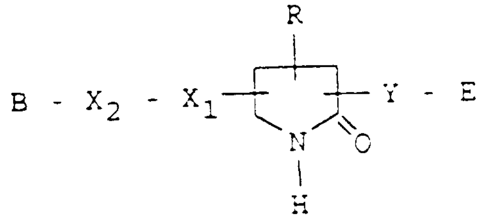

(I)

où, à condition que

(i) B représente un groupe $R_1$-CO-O-($R_2CR_3$)-O-CO-NH-C(=NH)-ou ($R_4$O)PO(O$R_5$)-NH-C(=NH)- ou E représente un groupe $R_6$-CO-O-CH$R_7$-O-CO- ou $R_8$O-CO- ou
(ii) B représente un groupe $R_1$-CO-O-($R_2CR_3$)-O-CO-NH-C(=NH)- ou ($R_4$O)PO(O$R_5$)-NH-C(=NH)- et E représente un groupe $R_6$-CO-O-CH$R_7$-O-CO- ou $R_8$O-CO-, dans lesquels

$R_1$ représente un groupe alkyle de 1 à 4 atomes de carbone,
$R_2$ représente un atome d'hydrogène ou un groupe méthyle,
$R_3$ représente un atome d'hydrogène,
$R_4$ et $R_5$, qui peuvent être identiques ou différents, représentent des groupes méthyle ou éthyle,
$R_6$ représente un groupe alkyle de 1 à 4 atomes de carbone, un groupe cyclohexyle ou phényle, un groupe alcoxy de 1 à 4 atomes de carbone ou un groupe cycloalcoxy de 5 à 7 atomes de carbone,
$R_7$ représente un atome d'hydrogène ou un groupe méthyle et
$R_8$ représente un groupe cycloalkyle, cycloalkylméthyle ou cycloalkyléthyle de 5 à 8 atomes de carbone dans la partie cycloalkyle dans chaque cas, les parties cycloalkyle citées précédemment pouvant être substituées en outre par un groupe alkyle de 1 à 4 atomes de carbone ou par un groupe alkyle de 1 à 4 atomes de carbone et 1 à 3 groupes méthyle, par un groupe méthoxy, éthoxy, diméthylamino ou diéthylamino et en outre dans les parties cycloalkyle citées précédemment dans chaque cas un groupe méthylène pouvant être remplacé par un atome d'oxygène, par un groupe méthylimino ou éthylimino à condition qu'il y ait au moins deux atomes de carbone entre l'hétéroatome cyclique et l'hétéroatome le plus proche,
un groupe cyclohéxényle ou cyclohéxénylméthyle, les parties cyclohéxényle citées précédemment pouvant être substituées en outre par un groupe méthyle, à condition que les parties cyclohéxényle citées précédemment ne soient pas liées à l'atome d'oxygène du groupe -O-CO- voisin par un atome de carbone duquel est issue une double liaison,
un groupe bicycloalkyle ou bicycloalkylalkyle de 6 à 8 atomes de carbone dans la partie bicycloalkyle et de 1 ou 2 atomes de carbone dans la partie alkyle dans chaque cas, les parties bicycloalkyle citées précédemment pouvant être substituées en outre par 1 à 3 groupes méthyle,
un groupe bicycloalcényle ou bicycloalcénylalkyle de 7 ou 8 atomes de carbone dans la partie bicycloalcényle et 1 ou 2 atomes de carbone dans la partie alkyle dans chaque cas, les parties bicycloalcényle citées précédemment pouvant en outre être substituées par 1 à 3 groupes méthyle, a condition que les parties bicycloalcényle citées précédemment ne soient pas liées à l'atome d'oxygène du groupe -O-CO- voisin par un atome de carbone duquel est issue une double liaison,

un groupe benzocycloalkyle de 9 ou 10 atomes de carbone au total,

un groupe alcényle ou alcynyle de 3 à 5 atomes de carbone dans chaque cas à condition que les groupes alcényle ou alcynyle cités précédemment ne soient pas liés à l'atome d'oxygène du groupe -O-CO- voisin par un atome de carbone duquel est issue une double ou triple liaison,

ou

un groupe cinnamyle,

R représente un atome d'hydrogène lié à un atome de carbone du cycle 2-pyrrolidinone ou un groupe méthyle,

B représente un groupe $HNR_{11}$-C(=NH)- dans lequel $R_{11}$ représente un atome d'hydrogène, un groupe méthoxycarbonyle, éthoxycarbonyle ou benzyloxycarbonyle, un groupe $R_1$-CO-O-($R_2CR_3$)-O-CO-NH-C(=NH)- ou ($R_4$O)PO($OR_5$)-NH-C(=NH)- dans lesquels $R_1$ à $R_5$ sont définis comme indiqué précédemment,

Y représente un groupe méthylène,

E représente un groupe carboxyle, un groupe alcoxycarbonyle de 2 à 6 atomes de carbone au total, un groupe $R_6$-CO-O-$CHR_7$-O-CO- ou $R_8$O-CO- où $R_6$ à $R_8$ sont définis comme indiqué précédemment,

$X_1$ représente un groupe méthylène qui, dans la mesure où il n'est pas lié à l'atome d'azote cyclique du cycle 2-pyrrolidinone, est lié par un atome d'oxygène, un groupe sulfonyle, -CO-NH- ou $SO_2$-NH- au groupe $X_2$ voisin, les groupes carbonyle et sulfonyle des groupes -CO-NH- et -$SO_2$-NH- cités précédemment étant liés au reste $X_2$, et

$X_2$ représente un groupe biphénylylène qui peut être substitué par un atome de fluor ou de chlore, par un groupe méthyle ou trifluorométhyle ou par un autre groupe méthyle,

leurs stéréoisomères, leurs tautomères, leurs mélanges et leurs sels.

2. Dérivés imino cycliques de formule générale I selon la revendication 1, dans lesquels, à condition que

(i) B représente un groupe $R_1$-CO-O-($R_2CR_3$)-O-CO-NH-C(=NH)-ou ($R_4$O)PO($OR_5$)-NH-C(=NH)- ou E représente un groupe $R_6$-CO-O-$CHR_7$-O-CO- ou $R_8$O-CO- ou

(ii) B représente un groupe $R_1$-CO-O-($R_2CR_3$)-O-CO-NH-C(=NH)-ou ($R_4$O)PO($OR_5$)-NH-C(=NH)- et E représente un groupe $R_6$-CO-O-$CHR_7$-O-CO- ou $R_8$O-CO-, dans lesquels

$R_1$ représente un groupe méthyle,

$R_2$ représente un atome d'hydrogène ou un groupe méthyle,

$R_3$ représente un atome d'hydrogène,

$R_4$ et $R_5$ représentent chacun un groupe éthyle,

$R_6$ représente un groupe tert.butyle, un groupe éthoxy ou cyclohexyloxy,

$R_7$ représente un atome d'hydrogène ou un groupe méthyle et

$R_8$ représente un groupe cyclopentyle, cyclohexyle, cycloheptyle ou cyclohexylméthyle,

R représente un atome d'hydrogène,

B représente un groupe $HNR_{11}$-C(=NH)- dans lequel $R_{11}$ représente un atome d'hydrogène, un groupe méthoxycarbonyle ou benzyloxycarbonyle, un groupe $R_1$-CO-O-($R_2CR_3$)-O-CO-NH-C(=NH)- ou ($R_4$O)PO($OR_5$)-NH-C(=NH)- dans lesquels $R_1$ à $R_5$ sont définis comme indiqué précédemment,

Y représente un groupe méthylène,

E représente un groupe méthoxycarbonyle, un groupe $R_6$-CO-O-$CHR_7$-O-CO- ou $R_8$O-CO- où $R_6$ à $R_8$ sont définis comme indiqué précédemment,

$X_1$ et $X_2$ représentent ensemble un groupe 4-biphénylylèneoxyméthylène, le groupe B étant lié en position 4' au groupe 4-biphénylylèneoxyméthylène, et le groupe méthylène du groupe 4-biphénylylèneoxyméthylène étant lié à un atome de carbone du cycle 2-pyrrolidinone,

leurs stéréoisomères, leurs tautomères, leurs mélanges et leurs sels.

3. Dérivés imino cycliques de formule générale I selon la revendication 1 dans lesquels A, B, E, $X_1$, $X_2$ et Y sont définis comme dans la revendication 2,

le reste -Y-E est situé en position 3 et

le reste B-$X_2$-$X_1$- est situé en position 5 du cycle 2-pyrrolidinone,

leurs stéréoisomères, leurs tautomères, leurs mélanges et leurs sels.

4. Composés de formule générale I selon la revendication 1 suivants:

(3S,5S)-5-[[4'-acétoxyméthyloxycarbonylamidino-4-biphénylyl]oxyméthyl]-3-[(méthoxycarbonyl)méthyl]-2-pyrrolidinone,

(3S,5S)-5-[[4'-(1-acétoxyéthyl)oxycarbonylamidino-4-biphénylyl]oxyméthyl]-3-[(méthoxycarbonyl)méthyl]-2-pyrrolidinone,

(3S,5S)-5-[(4'-benzyloxycarbonylamidino-4-biphénylyl]oxyméthyl]-3-[[[1-(cyclohexyloxycarbonyloxy)éthyl]oxycarbonyl]-méthyl]-2-pyrrolidinone,

(3S,5S)-5-[(4'-benzyloxycarbonylamidino-4-biphénylyl)oxyméthyl]-3-[[[1-(éthyloxycarbonyloxy)éthyl]oxycarbonyl]méthyl]-2-pyrrolidinone,

(3S,5S)-5-[(4'-benzyloxycarbonylamidino-4-biphénylyl)oxyméthyl]-3-[(pivaloyloxyméthyloxycarbonyl)méthyl]-2-pyrolidinone,

(3S,5S)-5-[(4'-méthoxycarbonylamidino-4-biphénylyl)oxyméthyl]-3-[(pivaloyloxyméthyloxycarbonyl)méthyl]-2-pyrrolidinone,

(3S,5S)-5-[(4'-amidino-4-biphénylyl)oxyméthyl]-3-[[[1-(cyclohexyloxycarbonyloxy)éthyl]oxycarbonyl]méthyl]-2-pyrrolidinone,

(3S,5S)-5-[(4'-amidino-4-biphénylyl)oxyméthyl]-3-[[[1-(éthyloxycarbonyloxy)éthyl]oxycarbonyl]méthyl]-2-pyrrolidinone,

(3S,5S)-5-[(4'-amidino-4-biphénylyl)oxyméthyl]-3-[(pivaloyloxyméthyloxycarbonyl)méthyl]-2-pyrrolidinone,

(3S,5S)-5-[(4'-amidino-4-biphénylyl)oxyméthyl]-3-[(cyclohexyloxycarbonyl)méthyl]-2-pyrrolidinone,

(3S,5S)-5-[(4'-amidino-4-biphénylyl)oxyméthyl]-3-[(cyclohexylméthyloxycarbonyl)méthyl]-2-pyrrolidinone,

(3S,5S)-5-[(4'-amidino-4-biphénylyl)oxyméthyl]-3-[(cyclopentyloxycarbonyl)méthyl]-2-pyrrolidinone,

(3S,5S)-5-[(4'-amidino-4-biphénylyl)oxyméthyl]-3-[(cycloheptyloxycarbonyl)méthyl]-2-pyrrolidinone,

(3S,5S)-3-[(cyclohexyloxycarbonyl)méthyl]-5-[(4'-méthoxycarbonylamidino-4-biphénylyl)oxyméthyl]-2-pyrrolidinone et

(3S,5S)-5-[[4'-(O,O'-Diéthylphosphono)amidino-4-biphénylyl]oxyméthyl]-3-[(méthoxycarbonyl)méthyl]-2-pyrrolidinone
ainsi que leurs sels.

5. Sels physiologiquement acceptables des composés selon au moins l'une des revendications 1 à 4 avec des acides ou des bases inorganiques ou organiques.

6. Médicament contenant un composé selon au moins l'une des revendications 1 à 4 ou un sel physiologiquement acceptable selon la revendication 5 outre éventuellement un ou plusieurs supports et/ou diluants inertes.

7. Utilisation d'un composé selon au moins l'une des revendications 1 à 5 pour la préparation d'un médicament qui convient pour la lutte contre ou la prévention de maladies dans lesquelles des agrégats cellulaires de taille relativement petite ou relativement grande apparaissent ou des interactions cellule-matrice jouent un rôle.

8. Procédé de préparation d'un médicament selon la revendication 6 caractérisé en ce que, par voie non chimique, un composé selon au moins l'une des revendications 1 à 5 est incorporé dans un ou plusieurs supports et/ou diluants inertes.

9. Procédé de préparation des dérivés imino cycliques selon les revendications 1 à 5 caractérisé en ce que

a) pour la préparation de composés de formule générale I où B représente un groupe $R_1$-CO-O-($R_2CR_3$)-O-

CO-NH-C(=NH)- ou $HNR_{11}$-C(=NH)-, où $R_1$ à $R_3$ sont définis comme dans les revendications 1 à 4, et $R_{11}$ représente un groupe méthoxycarbonyle, éthoxycarbonyle ou benzyloxycarbonyle, un composé de formule générale

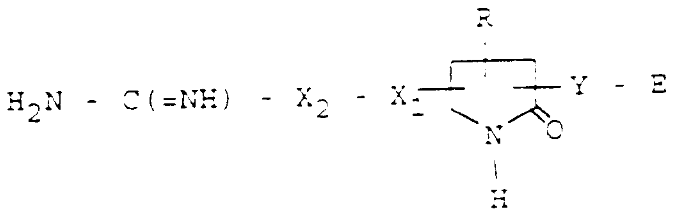

(II)

où

R, E, $X_1$, $X_2$ et Y sont définis comme dans les revendications 1 à 4 est mis à réagir avec un composé de formule générale

$$Z_1 - B_1 \qquad (III)$$

où
$B_1$ représente un groupe méthoxycarbonyle, éthoxycarbonyle ou benzyloxycarbonyle ou un groupe $R_1$-CO-O-($R_2CR_3$)-O-CO- dans lequel $R_1$ à $R_3$ sont définis comme dans les revendications 1 à 4, et
$Z_1$ représente un groupe partant nucléophile, ou

b) pour la préparation de composés de formule générale I où E représente un groupe alcoxycarbonyle de 2 à 6 atomes de carbone au total, un groupe $R_6$-CO-O-$CHR_7$-O-CO- ou $R_8$O-CO-, où $R_6$ à $R_8$ sont définis comme dans les revendications 1 à 4, un composé de formule générale

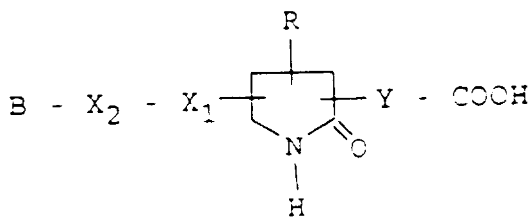

(IV)

où

R, B, $X_1$, $X_2$ et Y sont définis comme dans les revendications 1 à 4 est mis à réagir avec un composé de formule générale

$$Z_2 - E_1 \qquad (V)$$

où
$E_1$ représente un groupe alkyle de 1 à 5 atomes de carbone au total, un groupe $R_6$-CO-O-$CHR_7$- ou $R_8$-, où $R_6$ à $R_8$ sont définis comme dans les revendications 1 à 4, et
$Z_2$ représente un groupe partant nucléophile, ou

c) pour la préparation de composés de formule générale I où E représente un groupe alcoxycarbonyle de 2 à 6 atomes de carbone au total ou un groupe $R_8$O-CO- où $R_8$ est défini comme dans les revendications 1 à 4, un composé de formule générale

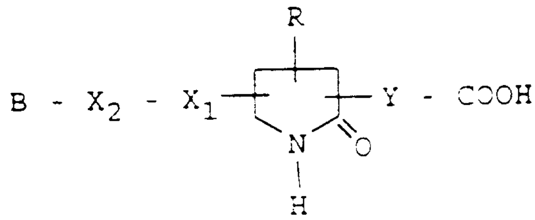

(IV)

où

R, B, $X_1$, $X_2$ et Y sont définis comme dans les revendications 1 à 4, est estérifié avec un composé de formule générale

$$HO - E_2 \qquad (VI)$$

où
$E_2$ représente un groupe alkyle de 1 à 5 atomes de carbone ou un groupe $R_8$- où $R_8$ est défini comme dans les revendications 1 à 4, ou

d) pour la préparation de composés de formule générale I où B représente un groupe $(R_4O)PO(OR_5)$-NH-C(=NH)- où $R_4$ et $R_5$ sont définis comme dans les revendications 1 à 4, un composé de formule générale

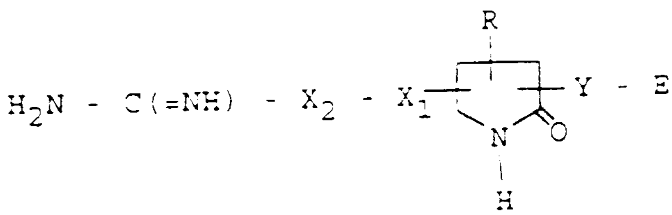

(II)

où

R, E, $X_1$, $X_2$ et Y sont définis comme dans les revendications 1 à 4, est mis à réagir avec un composé de formule générale

$$(R_4O)PO(OR_5)-Z_3 \qquad (VII)$$

où
$R_4$ et $R_5$ sont définis comme dans les revendications 1 à 4 et
$Z_3$ représente un groupe partant nucléophile, et

si nécessaire, un reste protecteur utilisé pendant les réactions pour la protection de groupes réactifs est clivé et/ou
si on le souhaite un composé de formule générale I ainsi obtenu est résolu en ses stéréoisomères et/ou
un composé de formule générale I ainsi obtenu est converti en ses sels, en particulier pour l'utilisation pharmaceutique en ses sels physiologiquement acceptables avec un acide ou une base inorganique ou organique.